(19) Europäisches Patentamt
European Patent Office
Office européen des brevets

(11) Publication number: **0 386 920 A2**

(12) # EUROPEAN PATENT APPLICATION

(21) Application number: 90302062.6

(22) Date of filing: 27.02.90

(51) Int. Cl.[5]: C07D 333/32, C07D 409/12, A61K 31/38

The title of the invention has been amended (Guidelines for Examination in the EPO, A-III, 7.3).

(30) Priority: 08.03.89 GB 8905336

(43) Date of publication of application: 12.09.90 Bulletin 90/37

(84) Designated Contracting States: AT BE CH DE DK ES FR GB GR IT LI LU NL SE

(71) Applicant: IMPERIAL CHEMICAL INDUSTRIES PLC
Imperial Chemical House, Millbank
London SW1P 3JF(GB)

(72) Inventor: Eakin, Murdoch Allan
2 Cockhall Lane, Langley
Macclesfield, Cheshire(GB)
Inventor: Cooper, Michael John
97 Buxton Road, Buglawton
Congleton, Cheshire(GB)

(74) Representative: Denerley, Paul Millington, Dr. et al
Imperial Chemical Industries PLC Legal Department: Patents P.O. Box 6, Bessemer Road
Welwyn Garden City Hertfordshire AL7 1HD(GB)

(54) **2-Hydroxy-3-thienyloxypropylamino derivatives.**

(57) 2-Hydroxy-3-thienyloxypropylamino compounds are described as useful in the treatment of obesity and related conditions. Processes for their preparation are described, as are novel intermediates and pharmaceutical compositions containing them.

EP 0 386 920 A2

# THIOPHENES

The present invention relates to 2-hydroxy-3-thienyloxypropylamino compounds and in particular to such compounds containing a phenoxymethyl group and derivatives thereof. This invention further relates to processes and intermediates for their preparation, to their use in methods of therapy and to pharmaceutical compositions containing them. Administration of the compounds of this invention to warm-blooded animals provides a thermogenic effect, that is thermogenesis is stimulated and administration of the compounds is of use, for example, in the treatment of obesity and related conditions such as obesity of mature onset diabetes.

In EP-A-140243 there is described a series of phenoxyacetic acid derivatives which are said to be of value in treating obesity. We have now discovered that, unexpectedly, the compounds of the present invention provide significant thermogenic properties at doses which cause relatively little cardiac stimulation and/or other side-effects such as lack of peripheral effect on the vasculature system. It is understood that selectivity of thermogenic effect is an important requirement for a useful therapeutic agent in the treatment of, for example, obesity and related conditions. The compounds of the present invention have a very desirable profile of activity.

Accordingly the present invention provides a compound of the formula (I):

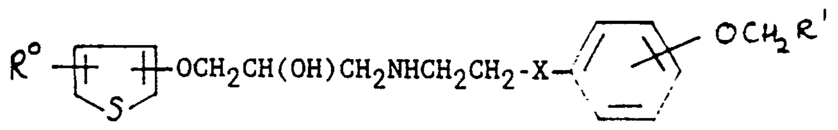

(I)

wherein

X is oxygen or is a bond;

$R^1$ is carboxy(-COOH), $C_{1-6}$alkoxycarbonyl, or a group $-CONR^2R^3$ wherein $R^2$ is hydrogen, $C_{1-6}$alkyl or $C_{3-6}$alkenyl (either group being optionally substituted by hydroxy, $C_{1-4}$alkoxy, phenoxy, carbamoyl, $C_{1-6}$alkylcarbamoyl, di-$C_{1-6}$alkylcarbamoyl, pyridyl, phenyl, chlorophenyl or chloropyridyl), $C_{3-6}$alkynyl, $C_{3-6}$cycloalkyl or $R^2$ is phenyl optionally substituted by halo, $C_{1-4}$alkyl, $C_{1-4}$alkoxy, trifluoromethyl, cyano or nitro;

$R^3$ is hydrogen, $C_{1-6}$alkyl, or a group $-OR^4$ wherein $R^4$ is hydrogen, $C_{1-6}$alkyl optionally substituted by hydroxy or $C_{1-4}$alkoxy, $C_{3-6}$alkenyl optionally substituted by hydroxy or $C_{1-4}$alkoxy, or $R^4$ is $C_{3-6}$alkynyl, phenyl, naphthyl, phenyl($C_{1-4}$)alkyl or naphthyl($C_{1-4}$)alkyl;

or $R^2$ and $R^3$ together form a $C_{4-7}$ methylene chain (wherein one methylene unit may optionally be replaced by oxygen or sulphur situated at least two carbon atoms distant from the nitrogen atom of $-NR^2R^3$) in which two adjacent methylene units may optionally be replaced by two carbon atoms of a benzene ring fused to said $C_{4-7}$ methylene chain said benzene ring optionally substituted by halo, $C_{1-4}$ alkyl, $C_{1-4}$ alkoxy, trifluoromethyl, cyano or nitro, or $R^2$ and $R^4$ together form a $C_{3-4}$ methylene chain;

or $R^1$ is $C_{1-6}$alkanoyl or a group $-(CH_2)_nOR^5$ wherein n is 1, 3 or 5 and $R^5$ is hydrogen, $C_{1-4}$alkyl or $C_{1-4}$alkanoyl;

and wherein $R^0$ is hydrogen or is chloro or fluoro substituted ortho to the aminopropoxy side-chain:

or a pharmaceutially acceptable salt thereof.

The $-OCH_2R^1$ substituent in the compounds of the formula (I) is generally situated in the meta or para position of the phenyl ring relative to the thienyloxypropylaminoethyl or thienyloxypropylaminoethoxy substituent. Preferably the substituents are in a para relationship.

The thiophene ring may carry the aminopropoxy side-chain in either the 2- or the 3-position. 2-Thienyl compounds may be optionally substituted in the 3-position by chloro or fluoro. 3-Thienyl compounds may be monosubstituted either in the 2- or 4-position by chloro or fluoro. It is preferred that the thiophene ring is unsubstituted, that is $R^0$ is hydrogen.

In one aspect X is oxygen so forming aminoethoxyphenyl compounds. In another aspect X is a bond so forming aminoethylphenyl compounds.

In a particular aspect $R^1$ is a group of the formula $-CONR^2R^3$. $R^2$ is hydrogen, $C_{1-6}$ alkyl for example methyl, ethyl, isopropyl, n-propyl, butyl, pentyl and hexyl or is $C_{3-6}$ alkenyl for example allyl. Optional substituents for $R^2$ being $C_{1-6}$ alkyl or $C_{3-6}$ alkenyl include hydroxy, $C_{1-4}$ alkoxy for example methoxy or ethoxy, phenyl, carbamoyl, $C_{1-6}$ alkylcarbamoyl for example methylcarbamoyl, di-$C_{1-6}$alkylcarbamoyl for example dimethylcarbamoyl and pyridyl.

An example of $R^2$ being $C_{3-6}$ alkynyl is propynyl.

$R^2$ can also be $C_{3-6}$ cycloalkyl that is cyclopropyl, cyclobutyl, cyclopentyl and cyclohexyl.

When $R^2$ is phenyl optional substituents are halo for example bromo, chloro and fluoro, $C_{1-4}$ alkyl for example methyl and ethyl, $C_{1-4}$ alkoxy for example methoxy and ethoxy, trifluoromethyl, cyano and nitro.

In one aspect $R^3$ is hydrogen or $C_{1-6}$ alkyl for example methyl or ethyl.

In a further aspect $R^3$ is a group $-OR^4$, thus forming a hydroxamic acid or derivative thereof. Preferably $R^4$ is hydrogen, $C_{1-6}$ alkyl optionally substituted by hydroxy or $C_{1-4}$ alkoxy for example methyl, ethyl, propyl, butyl, 2-hydroxyethyl, 3-hydroxypropyl, 2-methoxyethyl or 2-ethoxyethyl, or $R^4$ is $C_{3-6}$ alkenyl optionally substituted by hydroxy or $C_{1-4}$ alkoxy for example allyl, or $C_{3-6}$ alkynyl for example propynyl. In particular $R^4$ is hydrogen or $C_{1-6}$ alkyl.

In yet a further aspect $R^2$ and $R^3$ together form a $C_{4-7}$ methylene chain, so as to form, together with the nitrogen atom to which they are attached, a 5-8 membered heterocyclic ring. Optionally one methylene unit of the chain is replaced by oxygen or sulphur. For example $-NR^2R^3$ may represent pyrrolidino, piperidino, hexahydroazepino, morpholino or thiamorpholino. Any two adjacent methylene units of the chain may optionally be replaced by two carbon atoms of a benzene ring fused to said chain and said benzene ring may be optionally substituted by halo for example chloro, fluoro or bromo; $C_{1-4}$ alkyl for example methyl, ethyl or propyl; $C_{1-4}$ alkoxy for example methoxy or ethoxy; trifluoromethyl, cyano or nitro.

In another aspect $R^2$ and $R^4$ together form a $C_{3-4}$methylene chain, so that together with the -N-O- atoms the isooxazolidino or tetrahydroisooxazino moiety is formed.

In another aspect $R^1$ is carboxy or $C_{1-6}$alkoxycarbonyl. Preferred values of $R^1$ include carboxy, methoxycarbonyl, ethoxycarbonyl, n-propoxycarbonyl and t-butoxycarbonyl.

In a further aspect $R^1$ is $C_{1-6}$alkanoyl for example formyl, acetyl and propionyl.

In yet a further aspect $R^1$ is a group $-(CH_2)_nOR^5$ wherein n is 1, 3 or 5 and $R^5$ is hydrogen, $C_{1-4}$alkanoyl for example acetyl or propionyl, or $C_{1-4}$alkyl for example methyl, ethyl, propyl or butyl. Thus a specific value of $R^1$ is ethoxymethyl.

A preferred group of compounds of the present invention is that of the formula (II):

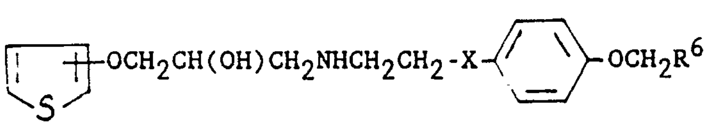

(II)

wherein X is oxygen or a bond and $R^6$ is carboxy, $C_{1-6}$alkoxycarbonyl, carbamoyl, $C_{1-6}$alkylcarbamoyl, hydroxy-$C_{1-6}$alkylcarbamoyl, $C_{1-4}$alkoxy$C_{1-6}$alkylcarbamoyl, di-$C_{1-6}$alkyl-carbamoyl, piperidino or morpholino.

In particular such compounds are preferred wherein the thiophene is substituted in the 3-position by the aminopropoxy side chain and X is an oxygen atom; that is compounds of the formula (IIA):

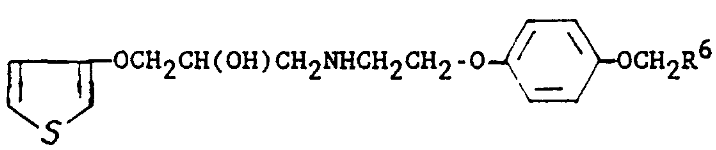

(IIA)

Particularly preferred values of $R^6$ in the compounds of the formula (II) and (IIA) are carboxy (-COOH), methoxycarbonyl (-COOMe), carbamoyl ($-CONH_2$), methylcarbamoyl (-CONHMe) and 2-methoxyethylcarbamoyl ($-CONHCH_2CH_2OMe$).

The compounds of the formula (I) are basic and may be isolated and used either in the form of the free base or of a pharmaceutically acceptable acid-addition salt thereof. In addition, certain compounds (e.g. wherein $R^1$ is carboxy) are amphoteric and may be used in the zwitterionic form, or as a pharmaceutically acceptable acid addition salt, or as a salt with a base affording a pharmaceutically acceptable cation. Particular examples of pharmaceutically acceptable acid-addition salts include, for example, salts with inorganic acids such as hydrohalides (especially hydrochlorides or hydrobromides), sulphates and phosphates, and salts with organic acids such as succinates, citrates, lactates, tartrates, oxalates and salts derived from acidic water-soluble polymers. Particular examples of salts with bases affording a pharmaceutically acceptable cation include, for example, alkali metal and alkaline earth metal salts, such as sodium, potassium, calcium and magnesium salts, and ammonium salts and salts with suitable organic bases such as triethanolamine.

It will be appreciated that the compounds of formula I contain one or more asymmetric carbon atoms and can exist as optically active enantiomers or as optically inactive racemates. The present invention encompasses any enantiomer, racemate and/or (when two or more asymmetric carbon atoms are present) diastereoisomer, which when administered in a therapeutic amount provides a thermogenic effect in warm-blooded animals, it being well known in the chemical art how to prepare individual enantiomers, for example by resolution of the racemate or by stereospecific synthesis, and how to determine the thermogenic properties, for example, using the standard tests described hereinafter. It is preferred that the compounds of the present invention are provided in the laevorotatory optically active form (-) which corresponds to (S) absolute configuration under the Cahn-Prelog-Ingold rules.

We believe, but do not wish to be bound by theory, that compounds of the formula (I) [other than those wherein $R^1$ is carboxy], in particular those wherein $R^1$ is $C_{1-6}$alkoxycarbonyl or a group $-CONR^2R^3$, at least in part, when administered to a warm-blooded animal form compounds of the formula (I) wherein $R^1$ is carboxy. Such compounds possess significant thermogenic properties with selectivity of effect. These compounds may be administered to a warm-blooded animal in an appropriate pharmaceutical composition as described herein, but preferably are formed in vivo from other compounds of the formula (I).

Specific compounds of the present invention include:
methyl 4-[2-(2-hydroxy-3-(3-thienyloxy)propylamino)ethyl]phenoxyacetate,
methyl 4-[2-(2-hydroxy-3-(3-thienyloxy)propylamino)ethoxy]phenoxyacetate,
(S)-methyl 4-[2-(2-hydroxy-3-(3-thienyloxy)propylamino)ethyl]phenoxyacetate,
(S)-methyl 4-[2-(2-hydroxy-3-(3-thienyloxy)propylamino)ethoxy]phenoxyacetate,
4-[2-(2-hydroxy-3-(3-thienyloxy)propylamino)ethoxy]phenoxyacetic acid,
4-[2-(2-hydroxy-3-(3-thienyloxy)propylamino)ethyl]phenoxyacetic acid,
(S)-4-[2-(2-hydroxy-3-(3-thienyloxy)propylamino)ethoxy]phenoxyacetic acid,
(S)-4-[2-(2-hydroxy-3-(3-thienyloxy)propylamino)ethyl]phenoxyacetic acid,
N-2-methoxyethyl-4-[2-(2-hydroxy-3-(3-thienyloxy)propylamino)ethoxy]phenoxyacetamide,
N-methyl-4-[2-(2-hydroxy-3-(3-thienyloxy)propylamino)ethoxy]phenoxyacetamide,
4-[2-(2-hydroxy-3-(3-thienyloxy)propylamino)ethoxy]phenoxyacetamide,
(S)-4-[2-(2-hydroxy-3-(3-thienyloxy)propylamino)ethoxy]phenoxyacetamide,
N-ethyl-4-[2-(2-hydroxy-3-(3-thienyloxy)propylamino)ethoxy]phenoxyacetamide and
(S)-N-methyl-4-[2-(2-hydroxy-3-(3-thienyloxy)propylamino)ethoxy]phenoxyacetamide.

In order to use a compound of the present invention or a pharmaceutically acceptable salt thereof for the therapeutic treatment of warm-blooded mammals including humans, in particular for treating obesity, it is normally formulated in accordance with standard pharmaceutical practice as a pharmaceutical composition.

Therefore in another aspect the present invention provides a pharmaceutical composition which comprises a compound of the formula (I) or a pharmaceutically acceptable salt thereof and a pharmaceutically acceptable carrier.

The pharmaceutical compositions of this invention may be administered in standard manner for example by oral or parenteral administration. For these purposes they may be formulated by means known to the art into the form of, for example, tablets, capsules, pills, powders, aqueous or oily solutions or suspensions, emulsions, and sterile injectable aqueous or oily solutions or suspensions.

In general compositions for oral administration are preferred.

The compositions may be obtained using standard excipients and procedures well known in the art. A unit dose form such as a tablet or capsule will usually contain, for example 0.1-250 mg of active ingredient. The compositions may also contain other active ingredients known for use in the treatment of obesity and related conditions, for example appetite suppressants, vitamins and hypoglycaemic agents.

In one aspect of the present invention, compounds of the formula (I) may be formulated for oral administration in a sustained (or delayed) release composition, for example a matrix tablet formulation comprising insoluble or swellable polymeric filler, or a coated spheroid formulation. In this manner compounds of the formula (I) are delivered, unchanged, to the gut whereupon they are released and subsequently pass into the bloodstream.

When used to produce thermogenic effects in warm-blooded animals including man, a compound of formula (I), or a pharmaceutically acceptable salt thereof as appropriate, will be administered so that a dose in the general range 0.002-20 mg/kg, and preferably in the range 0.02-10 mg/kg, is administered daily, given in a single dose or divided doses as necessary. However, it will be appreciated by those skilled in the art that dosage will necessarily be varied as appropriate, depending on the severity of the condition under treatment and on the age and sex of the patient and according to known medical principles.

In addition the compounds of the present invention lower triglyceride levels and cholesterol levels and

raise high density lipoprotein levels and are therefore of use in combatting medical conditions wherein such lowering (and raising) is thought to be beneficial. Thus they may be used in the treatment of hypertriglyceridaemia, hypercholesterolaemia and conditions of low HDL (high density lipoprotein) levels in addition to the treatment of atherosclerotic disease such as of coronary, cerebrovascular and peripheral arteries, cardiovascular disease and related conditions.

Accordingly in another aspect the present invention provides a method of lowering triglyceride and/or cholesterol levels and/or increasing high density lipoprotein levels which comprises administering, to an animal in need thereof, a therapeutically effective amount of a compound of the formula (I) or pharmaceutically acceptable salt thereof. In a further aspect the present invention provides a method of treating atherosclerosis which comprises administering, to an animal in need thereof, a therapeutically effective amount of a compound of the formula (I) or pharmaceutically acceptable salt thereof. The compositions are formulated and administered in the same general manner as detailed above for producing a thermogenic effect. They may also contain other active ingredients known for use in the treatment of atherosclerosis and related conditions, for example fibrates such as clofibrate, bezafibrate and gemfibrozil;
inhibitors of cholesterol biosynthesis such as HMG-CoA reductase inhibitors for example lovastatin, simvastatin and pravastatin;
inhibitors of cholesterol absorption for example beta-sitosterol and (acyl CoA:cholesterol acyltransferase) inhibitors for example melinamide; anion exchange resins for example cholestyramine, colestipol or a dialkylaminoalkyl derivatives of a cross-linked dextran; nicotinyl alcohol, nicotinic acid or a salt thereof; vitamin E; and thyromimetics.

In a further aspect the present invention provides a process for preparing a compound of the formula (I) or a pharmaceutically acceptable salt thereof which process comprises:
(a) reacting a compound of the formula (III) with a compound of the formula (IV):

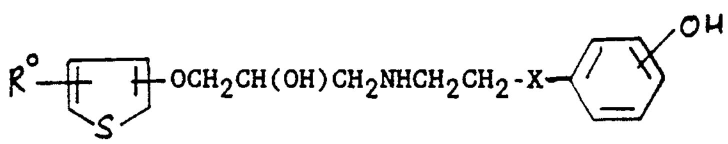

(III)

$L\text{-}CH_2\text{-}R^1$ (IV)

wherein X, $R^0$ and $R^1$ are as hereinabove defined and L is a displaceable group; or
(b) reacting a compound of the formula (V) with a compound of the formula (VI):

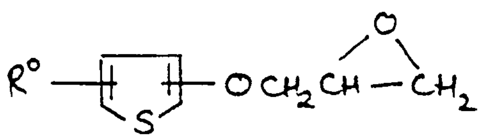

(V)

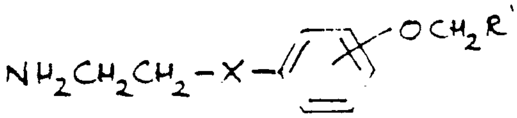

(VI)

wherein X, $R^0$ and $R^1$ are as hereinabove defined; or
(c) reducing a compound of the formula (VII):

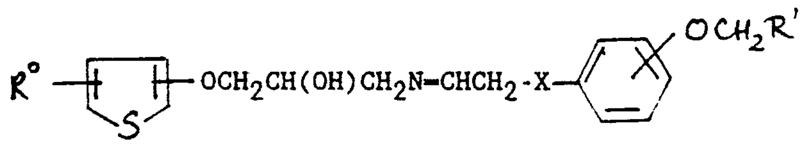

(VII)

wherein X, $R^0$ and $R^1$ are as hereinabove defined:

(d) for preparing compounds wherein $R^1$ is carbamoyl (-$CONH_2$), hydrolysing a compound of the formula (VIII):

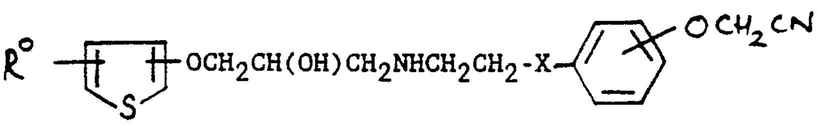

(VIII)

wherein X and $R^0$ are as hereinbefore defined:

and, if desired thereafter:

(i) converting a compound of the formula (I) into another compound of the formula (I), and/or

(ii) forming a pharmaceutically acceptable salt.

In the compounds of the formula (IV) L is a leaving group, for example chloro, iodo, bromo, methanesulphonyloxy or p-toluenesulphonyloxy. The reaction of the compounds of the formulae (III) and (IV) is conveniently performed in the presence of an external base, for example an inorganic base such as an alkali metal carbonate or acetate (for example potassium carbonate or sodium acetate), or an alkali metal hydride (e.g. sodium hydride), and at a temperature in the range, for example, 10 to 120 °C, conveniently at ambient temperature. A suitable solvent or diluent, for example acetone, methyl ethyl ketone, propan-2-ol, 1,2-dimethoxyethane or t-butyl methyl ether may conveniently be used. In order to minimise side-reactions, the process may also be carried out by pre-reacting the phenol of the formula (III) with a suitable base for example potassium tert-butoxide, to form the corresponding salt which is then added to the alkylating agent of the formula $LCH_2R^1$.

The compounds of the formula (III) may be obtained by conventional procedures of organic chemistry. Thus, for example, they may be obtained by reaction of a phenol of the formula (IX):-

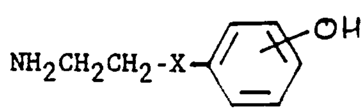

(IX)

wherein X is as hereinbefore defined with an epoxide of the formula (V) in a suitable solvent, for example, an alcohol such as ethanol or propan-2-ol, at a temperature in the range, for example, 10 ° to 110 °C and conveniently at or near the boiling point of the reaction mixture. The epoxides of the formula (V) are known from, or preparable by, the methods of Conde et al. European. J. Med. Chem, 18, 151 (1983). For example they can be made by reaction of a thiophene halide (e.g. bromide) with a sodium salt of protected glycerol, for example the acetone ketal (solketal) in the presence of copper oxide, followed by treatment with tosylate to form a 3-tosyloxy-2-hydroxypropoxythiophene which on subsequent treatment with base provides the epoxide. The above reaction sequence may be performed with racemic or optically active solketal.

The reaction of the compounds of the formulae (V) and (VI) is conveniently performed under conditions generally similar to those described hereinabove for the reaction between the compounds of the formulae (V) and (IX). When $R^1$ is alkoxycarbonyl there is the possibility of a competing self-condensation or polymerisation process of the compound of the formula (VI) so that this process is not preferred in such circumstances. The compounds of the formula (VI) may be prepared by reacting a compound of the formula (IX) with a compound of the formula (IV) under conditions generally similar to those described hereinabove for reacting compounds of the formulae (III) and (IV).

The reduction of a compound of the formula (VII) may be carried out using a reducing agent such as borohydride or a cyanoborohydride in a suitable polar solvent such as methanol or ethanol. The reaction may be performed at any convenient non-extreme temperature.

The compounds of the formula (VII) are conveniently formed by reacting a compound of the formula (X) with a compound of the formula (XI):

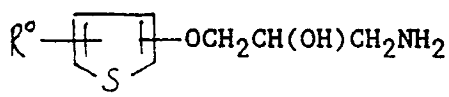

(X)

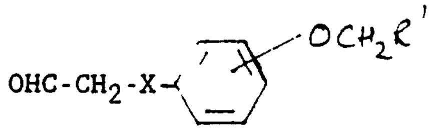

(XI)

wherein X, $R^0$ and $R^1$ are as hereinbefore defined. The reaction is typically performed at a non-extreme temperature in a substantially inert solvent for example a polar, aprotic solvent such as acetonitrile. Conveniently the compound of the formula (VII) is formed and reduced in situ. The compounds of the formula (X) are prepared by reacting ammonia, conveniently in methanolic solution, with a compound of the formula (V). The compounds of the formula (XI) may be prepared by reacting a compound of the formula (XII):

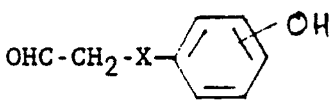

(XII)

wherein X is as hereinbefore defined with a compound of the formula (IV) under conditions generally similar to those described hereinabove for reacting compounds of the formulae (III) and (IV).

The compound of the formula (VIII) is conveniently hydrolysed under conditions standard for conversion of a nitrile to an amide for example with an alkali metal hydroxide such as sodium hydroxide under mild basic conditions. The compounds of the formula (VIII) are conveniently prepared by methods analogous to those described herein for compounds of the formula (I), for example by reacting a compound of the formula (III) and a compound of the formula $LCH_2CN$ wherein L is a leaving group.

Examples of converting one compound of the formula (I) into another compound of the formula (I) include the reaction of a compound wherein $R^1$ is $C_{1-6}$alkoxycarbonyl with a compound: $HNR^2R^3$. Such a reaction is generally performed in a suitable substantially inert solvent for example a $C_{1-4}$ alkanol such as methanol or ethanol, at a non-extreme temperature for example in the range 0-80°C. The reaction is optionally performed in a pressure vessel when a volatile amine is used. The compound $HNR^2R^3$ is conveniently present as an excess. Preferably in such a reaction $R^1$ is methoxycarbonyl or ethoxycarbonyl.

A reaction betweeen a suitably protected compound of the formula (I) wherein $R^1$ is carboxy or an activated derivative thereof and a compound $HNR^2R^3$ may be performed under conventional conditions, for example under standard acylation conditions wherein the acid is activated as the acid bromide, the acid chloride, an anhydride or activated ester, or the reaction is performed in the presence of a coupling agent such as dicyclohexylcarbodi-imide. The acid of the formula (I) is suitably protected prior to reaction, for example by silylation on the hydroxy group. Protecting groups can, of course, be removed in conventional manner upon completion of the acylation or coupling reaction. The acid of the formula (I) may be similarly reacted to give an ester of the formula (I) wherein $R^1$ is $C_{1-6}$alkoxycarbonyl.

In addition, compounds of the formula (I) wherein $R^1$ is carboxy may be prepared by the hydrolysis for example basic or enzymatic hydrolysis, of a corresponding ester or amide of the formula (I). Such hydrolysis may be performed under conventional conditions.

Suitable basic conditions may be used for example lithium, sodium or potassium hydroxide, conveniently in a suitable solvent or diluent such as an aqueous $C_{1-4}$alkanol at a temperature in the range, for example, 10° to 110°C. As yet further alternatives, when $R^1$ is t-butoxycarbonyl, the decomposition may be carried out, for example, by thermolysis at a temperature in the range, for example, 100 to 220°C, alone or in the presence of a suitable diluent such as diphenyl ether.

Furthermore, compounds of the formula (I) wherein $R^3$ is a group OR4 wherein $R^4$ is other than hydrogen may be prepared by reacting a compound of the formula (I) wherein $R^3$ is a group -OH with an alkylating agent (or arylating agent) under conventional alkylating conditions. For example, in general, O-alkylation is performed in the presence of an alkylating agent and about one equivalent of a suitable base. If $R^2$ is hydrogen, O,N-dialkylation is performed in the presence of an alkylating agent and about two equivalents of a suitable base. In particular a compound of the formula (I) wherein $R^2$ and $R^4$ together form

a $C_{3-4}$ methylene chain may be prepared by the alkylation of a compound of the formula (I) wherein $-NR^2R^3$ is -NHOH with a compound of the formula: $L^1(CH_2)_nL^2$ wherein n is 3 or 4 and $L^1$ and $L^2$ are both leaving groups.

Pharmaceutically acceptable salts may be prepared by reacting the compound of the formula (I) with the appropriate acid in conventional manner. Alternatively when a hydrogen halide salt is required, it may conveniently be obtained by hydrogenation of the free base together with a stoichiometric amount of the corresponding benzyl halide.

The following biological test methods, data and Examples serve to illustrate this invention.

The thermogenic effects of the compounds of the formula (I) may be demonstrated using one or more of the following standard tests:-

(a) Rats are cold adapted at 4° C for 4 days to increase their capacity for thermogenesis. They are then transferred to a warm environment of 23° C for 2 days. On the following day, a test compound is administered sub-cutaneously or orally as described in (a). Animals are sacrificed one hour later and the interscapular, brown adipose tissue (BAT) pad is removed. BAT mitochondria are prepared by differential centrifugation and GDP binding is determined (Holloway et al., International Journal of Obesity, 1984, 8, 295) as a measure of thermogenic activation. Each test includes a control which is dosed with the solution/suspension vehicle only and a positive control which is dosed with isoprenaline (as its sulphate) at 1 mg/kg. Test compounds are routinely dosed at 0.1, 0.3, 1.0, 3.0, and 10 mg/kg and results expressed in terms of the effect on GDP binding produced by isoprenaline. From these results, a dose ($ED_{50}$) necessary to produce 50% of the isoprenaline effect is calculated by linear regression analysis. Compounds are considered active in this test if they cause a significant elevation in GDP binding as compared to controls.

(b) Rats are adapted to a thermoneutral environment (29° C) for 2 weeks in order to decrease their capacity for BAT mediated non-shivering thermogenesis. During the final 3 days the animals are trained to use an apparatus for measuring heart rate non-invasively via foot-pad electrodes connected to an ECG integrator giving a continuous read-out of heart rate. A test compound is administered sub-cutaneously or orally at the $ED_{50}$ determined in test (a), and heart rate is determined 15-30 minutes after dosing. The procedure is then repeated in subsequent tests using increasing multiples of the $ED_{50}$ determined in test (a) until the heart rate (HR) reaches or exceeds 500 beats per minute, allowing the dose necessary to produce a heart rate of 500 beats per minute ($D_{500}$ dose) to be calculated. The ratio of $D_{500}$ to $ED_{50}$ in test (a) can be defined as the selectivity index (SI) and provides a measure of the selectivity of the compound for BAT as opposed to the cardiovascular system. Compounds are considered to have significant selectivity which have an SI of >1. Non-selective compounds have an SI of <1 (for example isoprenaline = 0.06).

(c) Rats are cold adapted at 4° C for four days to increase their capacity for thermogenesis. They are then transferred to a warm environment at 23° C for two days. On the following day, the basal metabolic rate of the animals is determined using a close-circuit oxygen consumption apparatus of the type described by Arundel et al., 1984, J. Appl. Physiol. Respirat. Environ. Exercise Physiol., 1984, 57 (5) 1591-1593. The rats are then dosed (orally or sub-cutaneously) with test compound at about (10 mg/kg) as a solution or suspension in 0.45% w/v aqueous sodium chloride, 0.25% w/v Polysorbate 80. Metabolic rate is then determined for at least one hour after dosing. Compounds are considered active in this test if they cause a significant increase in metabolic rate as compared to control animals (Student's t test: $p < 0.5$) dosed only the solution or suspension vehicle.

In the above tests, the compounds of the formula (I) in general produce effects of the following order without producing overt toxicity:-

test (a): sub-cutaneous or oral $ED_{50}$ for GDP binding in BAT mitochondria of 0.01-10 mg/kg;

test (b): show an SI of >50; and

test (c): show an increase of 2ml $O_2min^{-1}kg^{0.75}$ at $1mgkg^{-1}$ p.o. By way of illustration, the compound described in the accompanying Example 1, produced the following effects in the above tests:-

(a) sub-cutaneous (s.c) $ED_{50}$ 0.2 mg/kg;

(b) $D_{500}$: > 20 mg/kg (s.c); SI > 100 (s.c).

(c) an increase of 5.2 ml $O_2$ $min^{-1}Kg^{0.75}$ at $1mgkg^{-1}$ p.o.

The invention will now be illustrated by the following Examples in which, unless otherwise stated:

a) nuclear magnetic resonance (NMR) spectra were determined at 200MHz unless otherwise stated, in $d_6$-dimethylsulphoxide/$d_4$-acetic acid as solvent using tetramethylsilane (TMS) as an internal standard and are expressed in delta values (parts per million) for protons relative to TMS, using conventional abbreviations to describe signal types.

b) all crystalline end-products had satisfactory microanalyses.

c) chromatography was performed on Merck Kieselgel (art 7734) obtainable from E. Merck, Darmstadt, Federal Republic of Germany.

d) where noted, solutions were taken to low pH as judged by moist indicator paper.
e) evaporations were carried out under reduced pressure using a rotary evaporator.
f) melting-points were performed on a Buchi apparatus and were uncorrected.

**Example 1**

Methyl 4-[2-(2-hydroxy-3-(3-thienyloxy)propylamino)ethyl]phenoxyacetate.

Potassium t-butoxide (0.55g) was added to a solution of 4-[2-(2-hydroxy-3-(3-thienyloxy)propylamino)-ethyl]phenol (1.3g) in dimethoxyethane (70ml) and the mixture was stirred for 10 minutes. Methyl bromoacetate (0.7g) was added and the mixture stirred for 16 hours. The solvent was evaporated and the residue (2.2g) was purified by chromatography on K60 silica (80g), using 6% methanol in dichloromethane as eluant. The product was obtained as an oil (0.5g) which began to crystallise. It was dissolved in dichloromethane (50ml) and acidified to pH3 with ethereal hydrogen chloride. The solvent was evaporated to give a residue which was crystallised from methyl acetate (120ml) and methanol (20ml) to give methyl 4-[2-(2-hydroxy-3-(3-thienyloxy)propylamino)ethyl]phenoxyacetate hydrochloride (0.32g) m.p. 161-63°C; NMR: 2.87-3.03[m,2H, $CH_2C_6H_4$]; 3.03-3.28[m,4H,$CH_2NHCH_2$]; 3.72[s,3H,$CH_3$]; 3.95-4.05[m,2H, $OCH_2CHOH$]; 4.22[m,1H,CHOH]; 4.74[s,2H,$OCH_2CO$]; 6.56[m,1H,$H_2$ thienyl]; 6.78[m,1H,$H_4$ thienyl]; 6.85-7.25[m,4H,$C_6H_4$]; 7.38[m,1H,$H_5$ thienyl]; microanalysis: found: C, 53.6; H, 6.0; N, 3.5; Cl 8.8%; $C_{18}H_{23}NO_5S$. HCl requires: C, 53.8; H, 6.0; N, 3.5; Cl, 8.8%.

The starting material was prepared as follows:-

A mixture of 3-thienylglycidyl ether (1.0g) and tyramine (2.0g) in propan-2-ol (30ml) was heated to reflux for 2 hours. The solvent was evaporated and the residue was purified by chromatography on K60 silica (80g) using methanol in dichloromethane as eluant (10% rising to 12% methanol). Thus was obtained as an oil, which readily crystallised: 4-[2-(2-hydroxy-3-(3-thienyloxy)propylamino)ethyl]phenol (1.3g) NMR: 2.80-2.98[m,2H,$CH_2C_6H_4$]; 3.00-3.50[m,4H, $CH_2NHCH_2$]; 3.92-4.05[m,2H,$OCH_2CHOH$]; 4.22[m,1H,CHOH]; 6.53-[m,1H,$H_2$ thienyl]; 6.78[m,1H,$H_4$ thienyl]; 6.70-7.10[m,4H,$C_6H_4$]; 7.35[m,1H,$H_5$ thienyl]; M/S: CI($NH_3$) 294-$(M+H)^+$.

**Example 2**

Methyl 4-[2-(2-hydroxy-3-(3-thienyloxy)propylamino)ethoxy]phenoxyacetate.

Potassium t-butoxide (0.53g) was added to a solution of 4- [2-(2-hydroxy-3-(3-thienyloxy)propylamino)-ethoxy]phenol (1.27g) in dimethoxyethane (100ml) and the mixture was stirred for 10 minutes. Methyl bromoacetate (0.65g) was added and the mixture was stirred for 16 hours. The mixture was filtered through diatomaceous earth using methanol as eluant to remove some of the inorganic material. The solvent was removed by evaporation and the residue (2.5g) was purified on a column of K60 silica (75g) using 8% methanol in dichloromethane as eluant. A colourless solid was obtained (1.4g) which was dissolved in dichloromethane and acidified to pH3 with ethereal hydrogen chloride. The solvent was evaporated and crystallised from methyl acetate (20ml) and methanol (15ml) to give methyl 4-[2-(2-hydroxy-3-(3-thienyloxy)-propylamino)ethoxy] phenoxyacetate hydrochloride (0.8g): m.p. 156-58°C; NMR: 3.12-3.39[m,2H, $CHOHCH_2NH$]; 3.45[t,2H,$NHCH_2CH_2O$]; 3.73[s,3H,$CH_3$]; 3.95-4.10[m,2H,$OCH_2CHOH$]; 4.22-4.38-[m,3H,CHOH plus $NHCH_2CH_2O$]; 4.68[s,2H, $OCH_2CO$]; 6.53[m,1H,$H_2$ thienyl]; 6.79[m,1H,$H_4$ thienyl]; 6.85-7.0 [m,4H,$C_6H_4$]; 7.36[m,1H,$H_5$ thienyl]; microanalysis; found: C,51.7; H,5.5; N,3.1; S,7.7; Cl, 8.5%; $C_{18}H_{23}NO_6S$. HCl requires: C, 51.7; N, 5.8; N, 3.4; S, 7.7; Cl, 8.5%.

The starting material was prepared as follows:

A mixture of 3-thienylglycidyl ether (2.6g) and 4-(2-aminoethoxy)phenol (2.9g) in propan-2-ol (100ml) was heated to reflux for 2 hours. The solvent was evaporated and the residue (5.0g) was purified by chromatography on K60 silica (75g) using methanol in dichloromethane as eluant (8% increasing to 12% methanol) to give 4-[2-(2-hydroxy-3-(3-thienyloxy)propylamino)ethoxy]phenol (2.6g) as a white solid m.p. 138-40°C; NMR: 3.05-3.35[4d,2H, $CHOHCH_2N$]; 3.40[t,2H,$NHCH_2CH_2O$]; 3.98[d,2H,$OCH_2CHOH$]; 4.13-4.33-[m,3H, CHOH plus $CH_2OC_6H_4$]; 6.56[m,1H,$H_2$ thienyl]; 6.68-6.88[m,5H,$C_6H_4$ plus $H_4$ thienyl]; 7.40[m,1H,$H_5$ thienyl]; M/S: +FAB 310$(M+H)^+$.

## Example 3

Methyl 4-[2-(2-hydroxy-3-(2-thienyloxy)propylamino)ethoxy]phenoxyacetate.

Potassium t-butoxide (0.55g) was added to 4-[2-(2-hydroxy-3-(2-thienyloxy)propylamino)ethoxy]phenol (1.37g) in dimethoxyethane (100ml) and the mixture stirred for 10 minutes. Methyl bromoacetate (0.65g) was added and the mixture was stirred for a further 16 hours. It was filtered through diatomaceous earth washing with a little methanol. The filtrate was evaporated to give a brown oil (2.2g) which was purified by chromatography on K60 silica (70g) using 8% methanol in dichloromethane as eluant. The product from the chromatography was dissolved in dichloromethane (80ml) and acidified to pH3 with ethereal hydrogen chloride. The solvent was evaporated to give a colourless solid (1.0g) which was recrystallised from methyl acetate/methanol to give methyl 4-[2-(2-hydroxy-3-(2-thienyloxy)propylamino)ethoxy]phenoxyacetate hydrochloride (0.32g) m.p. 144-46° C; NMR: 3.02-3.35[4d,2H,$CHOHCH_2NH$]; 3.41[t,2H,$NHCH_2CH_20$]; 3.70-[s,3H,$OCH_3$]; 4.06[d,2H,$OCH_2CHOH$]; 4.25[m,3H,$NHCH_2CH_20$ plus CHOH]; 4.72[s,2H,$OCH_2CO$]; 6.35-[m,1H,$H_3$ thienyl]; 6.74[m,2H,$H_4$/$H_5$ thienyl]; 6.86-7.02[m,4H,$C_6H_4$]: microanalysis: found: C,51.6; H,5.7; N,3.5; S,7.8; Cl, 8.7%; $C_{18}H_{23}N0_6S$.HCl requires: C, 51.7; H, 5.8: N, 3.4; S, 7.7; Cl, 8.5%.

The starting material was prepared as follows:

A mixture of 2-thienylglycidyl ether (1.42g) and 4-(2-aminoethoxy)phenol (1.74g) in propan-2-ol (60ml) was heated to reflux for 2 hours. The mixture was evaporated and the residue purified by chromatography on K60 silica (70g) using methanol in dichloromethane as eluant (8% increasing to 12% methanol). An oil (1.45g) was obtained which solidified readily to give 4-[2-(2-hydroxy-3-(2-thienyloxy)propylamino)ethoxy]-phenol m.p. 125-7° C: NMR: 3.07-3.35[4d,2H,$CHOHCH_2NH$]; 3.42[t,2H,$NHCH_2CH_20$]; 4.08-[d,2H,$OCH_2CHOH$]; 4.21[t,2H, $NHCH_2CH_20$]; 4.28[m,1H,CHOH]; 6.33[m,1H,$H_3$ thienyl]; 6.65-6.88[m,6H,$C_6H_4$ plus $H_4$/$H_5$ thienyl]; M/S : 310$(M+H)^+$.

## Example 4

Methyl 4-[2-(2-hydroxy-3-(2-thienyloxy)propylamino)ethyl]phenoxyacetate.

Potassium t-butoxide (0.398g) was added to a solution of 4-[2-(2-hydroxy-3-(2-thienyloxy)propylamino)-ethyl]phenol (0.95g) in dimethoxyethane (70ml) and the mixture was stirred for 10 minutes before methyl bromoacetate was added. The mixture was stirred for 16 hours and was then evaporated to give a solid residue which was purified on a column of K60 silica (70g) using 6% methanol in dichloromethane as eluant. The product was collected as an oil (0.7g). The oil was dissolved in dichloromethane (100ml) and acidified to pH3 with ethereal hydrogen chloride. Evaporation of the solvent gave a white solid (0.75g) which was crystallised from methyl acetate/methanol/ether to give methyl 4-[2-(2-hydroxy-3-(2-thienyloxy)-propylamino)ethyl]phenoxyacetate hydrochloride (0.5g) m.p. 131-33° C: NMR: 2.93-3.32-[m,6H,$CH_2NHCH_2CH_2$]; 3.77[s,3H,$OCH_3$]; 4.11[d,2H,$OCH_2CHOH$]; 4.27[m,1H,CHOH]; 4.81[s,2H,$OCH_2CO$]; 6.39[m,1H,$H_3$ thienyl]; 6.78[m,2H,$H_4$/$H_5$ thienyl]; 6.92-7.30[m,4H,$C_6H_4$]; microanalysis: found: C, 53.2; H, 6.1; N, 3.5; S, 8.0; Cl, 8.5: $H_2O$; 0.1%; $C_{18}H_{23}NO_5S$. HCl. $0.1H_20$ requires: C, 53.6; H, 6.0; N, 3.5; S, 7.9; Cl, 8.5; $H_20$, 0.4%.

The starting material was prepared as follows:

A mixture of 2-thienylglycidyl ether (0.9g) and tyramine (2.0g) in propan-2-ol (30ml) was heated to reflux for 2 hours. The solvent was removed by evaporation under reduced pressure and the residue was purified by chromatography on K60 silica (80g) using 10% methanol in dichloromethane as eluant. An oil was obtained which readily crystallised to give 4-(2-[2-hydroxy-3-[2-thienyloxy]propyl)amino]ethyl)phenol (0.95g) m.p. 106-8° C: NMR: 2.80-2.95[m,2H,$CH_2C_6H_4$]; 2.95-3.25[m,4H,$CH_2NHCH_2$]; 4.05[d,2H,$OCH_2CHOH$]; 4.20-[m,1H,CHOH]; 6.34[m,1H,$H_3$ thienyl]; 6.72[m,2H,$H_4$/$H_5$ thienyl]; 6.75-7.10[m,4H,$C_6H_4$]: M/S 294$(M+H)^+$.

## Example 5

N-2-Methoxyethyl-4-[2-(2-hydroxy-3-(2-thienyloxy)propylamino)ethoxy]phenoxyacetamide.

A solution of methyl 4-[2-(2-hydroxy-3-(2-thienyloxy)propylamino)ethoxy]phenoxyacetate hydrochloride (0.6g) and 2-methoxyethylamine (4ml) in methanol (15ml) was kept at room temperature for 4 hours. The mixture was evaporated under reduced pressure (the last traces being co-evaporated with toluene) to give a residue (0.9g) which was purified by column chromatography on K60 silica (75g) using 10% methanol in dichloromethane as eluant. The desired product from the column was collected as an oil, dissolved in dichloromethane (50ml) and acidified to pH3 with ethereal hydrogen chloride. The solvent was evaporated to give a residue which was crystallised from methyl acetate/methanol/ether to give N-2-methoxyethyl-4-[2-(2-hydroxy-3-(2-thienyloxy)propylamino)ethoxy]phenoxyacetamide hydrochloride (0.35g) m.p. 145-47 °C; NMR: 3.07-3.50[m,11H,$CH_2NHCH_2$ plus $CH_2CH_2OCH_3$]; 4.08[d,2H,$OCH_2CHOH$]; 4.20-4.38[m, 3H, CHOH plus $CH_2OC_6H_4$]; 4.45[s,2H,$OCH_2CO$]; 6.34[m,1H,$H_3$ thienyl]; 6.65-6.78[m,2H,$H_4$/$H_5$ thienyl]; 6.94-[s,4H,$C_6H_4$]; microanalysis: found: C, 51.4; H, 6.3; N, 6.0; S, 6.6; Cl, 7.6; $H_2O$, 0.3%; $C_{20}H_{28}N_2O_6S$.HCl. 0.25 $H_2O$ requires: C, 51.6; H, 6.3; N, 6.3; S, 6.9; Cl, 7.6; $H_2O$, 1.0%:

## Example 6

N-2-Methoxyethyl-4-[2-(2-hydroxy-3-(3-thienyloxy)propylamino)ethoxy]phenoxyacetamide.

A solution of 3-thienylglycidyl ether (1.6g) and N-2-methoxyethyl-4-[2-aminoethoxy]phenoxyacetamide (3.0g) in propan-2-ol (20ml) and methanol (4ml) was maintained at room temperature for 40 hours. The solvent was evaporated and the residue was purified by chromatography on K60 silica (65g) using 6% methanol in dichloromethane as eluant. The product was obtained as a solid (2.0g), a sample of which (1.0g) was dissolved in dichloromethane and methanol and acidified to pH3 with ethereal hydrogen chloride. The solvent was evaporated to give a residue which was crystallised from methanol to give N-2-methoxyethyl-4-[2-(2-hydroxy-3-(3-thienyloxy)propylamino)ethoxy]phenoxyacetamide hydrochloride (0.7g) m.p. 172-73.5 °C; NMR: 3.07-3.50[m,8H,$CH_2NHCH_2$ plus $NHCH_2CH_2OCH_3$]; 3.28[s,3H,$OCH_3$]; 4.01-[m,2H,$OCH_2CHOH$]; 4.28[m,3H,CHOH and $CH_2CH_2OC_6H_4$]; 4.46[s,2H,$OCH_2CO$]; 6.55[m,1H,$H_2$ thienyl]; 6.79[m,1H,$H_4$ thienyl]; 6.95[s,4H,$C_6H_4$]; 7.37[m,1H,$H_5$ thienyl]; microanalysis: found: C, 51.7; H, 6.3; N, 5.9; S, 6.9; Cl, 7.9; $H_2O$, 0.3%; $C_{20}H_{28}N_2O_6S$.HCl. 0.1$H_2O$ requires: C, 51.9; H, 6.3; N, 6.1; S, 6.9; Cl, 7.7; $H_2O$ 0.4%.

N-2-Methoxyethyl-4-[2-aminoethoxy]phenoxyacetamide was obtained as follows:

N-2-Methoxyethyl-4-[2-(benzylamino)ethoxy]phenoxyacetamide (3.58g) was dissolved in propan-2-ol (160ml) and methanol (50ml). 10% Palladium on carbon was added and the mixture was stirred under an atmosphere of hydrogen for 24 hours. The mixture was filtered through Kieselguhr and the filtrate was evaporated to give N-2-methoxyethyl-4-(2-aminoethoxy)phenoxyacetamide (3.0g) as an oil, which was used without further purification.

Mass spectrum: +FAB 269$(M+H)^+$.

N-2-Methoxyethyl-4-[2-(benzylamino)ethoxy]phenoxyacetamide was prepared as follows:

Potassium t-butoxide (1.2 equivalents) in tetrahydrofuran was added to 4-[2-(N-benzylamino)ethoxy]-phenol (1 equivalent) suspended in tetrahydrofuran under an argon atmosphere. The temperature was maintained at 15-20 °C during the addition and the mixture was stirred for one hour. To this solution was added N-(2-methoxyethyl)chloroacetamide (1.1 equivalents) in tetrahydrofuran over 15 minutes. The resultant mixture was stirred for 5 hours, an approximately equal volume of water was added and tetrahydrofuran removed by distillation at 40 °C on a rotary evaporator. The aqueous solution was extracted into ethyl acetate (twice) and the combined ethyl acetate extracts were evaporated under reduced pressure to give as a solid, N-2-methoxyethyl-4-[2-(benzylamino)ethoxy]phenoxyacetamide which was crystallised from methyl t-butyl ether (yield 90%).

## Example 7

N,N-Diethyl-4-[2-(2-hydroxy-3-(3-thienyloxy)propylamino) ethyl]phenoxyacetamide.

A solution of 3-thienylglycidyl ether (1.5g) and N,N-diethyl-4-[2-aminoethyl]phenoxyacetamide (2.75g) in propan-2-ol was kept at ambient temperature for 72 hours. The solvent was evaporated and the residual gum (4.3g) was purified by chromatography on K60 silica (150g) using methanol in dichloromethane (4%

increasing to 5% methanol) as eluant. The product (1.4g) was dissolved in dichloromethane and acidified to pH3 with ethereal hydrogen chloride. The solvent was removed by evaporation to give a gum which was dissolved in methyl acetate, from which it crystallised. Recrystallisation from methyl acetate/methanol gave N,N-diethyl-4-[2-(2-hydroxy-3-(3-thienyloxy)propylamino]ethyl]phenoxyacetamide hydrochloride (0.8g) m.p. 121-23°C; NMR (DMSO-$d_6$): 1.04[t,3H,$CH_3$]; 1.14[t,3H, $CH_3$]; 2.85-3.40[m,10H, $CH_2NHCH_2CH_2$ plus $CH_3CH_2NCH_2CH_3$]; 3.95[m,2H, $OCH_2CHOH$]; 4.24[m,1H,CHOH]; 4.73[s,2H,$OCH_2CO$]; 5.91[d,1H,CHOH]; 6.63[m,1H,$H_2$ thienyl]; 6.80[m,1H,$H_4$ thienyl]; 6.83-7.23[m,4H,$C_6H_4$]; 7.43[m,1H,$H_5$ thienyl]: microanalysis: found: C, 53.6; H, 6.0; N, 3.5; Cl, 8.8%; $C_{18}H_{23}NO_5S$.HCl requires: C, 53.8; H, 6.0; N, 3.5; Cl, 8.8%.

N,N-Diethyl-4-[2-aminoethyl]phenoxyacetamide was obtained as follows:

N-Benzyltyramine (4.54g) was added to a stirred suspension of potassium t-butoxide (2.44g) in dimethoxyethane (150ml). A clear solution was produced which was stirred at room temperature for 10 minutes. N,N-Diethylchloroacetamide (3.0g) was added and the mixture was stirred for 1 hour. The mixture was evaporated and the residue dissolved in methanol and filtered through Kieselguhr. The filtrate was evaporated to give an oil which was partitioned between dichloromethane and water. The organic solution was dried over magnesium sulphate and evaporated to give N,N-diethyl-4-[2-(benzylamino)ethyl]-phenoxyacetamide (7.0g) as a yellow oil. This was dissolved in propan-2-ol (150ml) and 10% palladium on carbon was added. The mixture was stirred for 16 hours under an atmosphere of hydrogen, filtered through Kieselguhr and the solvent was evaporated to give N,N-diethyl-4-(2-aminoethyl)phenoxyacetamide (5.5g) as an oil, which was used directly, without further purification. Mass spectrum: 251 $(M+H)^+$.

## Example 8

### 4-[2-(2-Hydroxy-3-(3-thienyloxy)propylamino)ethyl]phenoxyacetic acid

To a solution of methyl 4-[2-(2-hydroxy-3-(3-thienyloxy)propylamino)ethyl]phenoxyacetate hydrochloride (0.85 g) in ethanol (65 ml) was added a solution of sodium hydroxide (0.3 g) in water (2 ml). The mixture was maintained at ambient temperature for 2 hours. The crude product was collected by filtration, washed with ethanol and dissolved in water (10 ml). The pH was reduced to 4 by the dropwise addition of 2N hydrochloric acid. A gum resulted which readily solidified. This solid was collected by filtration and dried at 60°C over $P_2O_5$ (in vacuo) to give 4-[2-(2-hydroxy-3-(3-thienyloxy)propylamino)ethyl]phenoxyacetic acid, (96% zwitterion 4% hydrochloride salt, 0.1 $H_2O$) (0.28 g) m.p. 170-75°C; NMR: 2.83-3.0[m, 2H, $CH_2C_6H_4$]; 3.0-3.28[m, 4H, $CH_2NHCH_2$]; 3.88-4.04 [d, 2H, $OCH_2CHOH$]; 4.18[m, 1H, CHOH]; 4.56[s, 2H, $OCH_2CO$]; 6.58[m, 1H, $H_2$ thienyl]; 6.80[m, 1H, $H_4$ thienyl]; 6.85- 7.25[m, 4H, $C_6H_4$]; 7.40[m, 1H, $H_5$ thienyl]: microanalysis: found C, 57.7; H, 6.0; N, 3.8; S, 9.0; Cl, 0.5; $H_2O$, 0.6%; $C_{17}H_{21}NO_5S$. 0.04HCl 0.1$H_2O$ requires: C, 57.6; H, 6.0; N, 4.0; S, 9.0; Cl, 0.4; $H_2O$, 0.5%.

## Example 9

### 4-[2-(2-Hydroxy-3-(3-thienyloxy)propylamino)ethoxy]phenoxyacetic acid.

To a solution of methyl 4-[2-(2-hydroxy-3-(3-thienyloxy)propylamino)ethoxy]phenoxyacetate (1.25 g) in ethanol (100 ml) was added a solution of potassium hydroxide (0.33 g) in water (3 ml). The solution was maintained at ambient temperature for 2 hours, subsequently solvent was removed by evaporation and the residue dissolved in water (150 ml). The resulting solution was acidified to pH 5.5 with perchloric acid (1M). The precipitate which formed was collected by filtration, washed with distilled water, and dried at 60°C under vacuo (over $P_2O_5$), to give 4-[2-(2-hydroxy-3-(3-thienyloxy)propylamino)ethoxy]phenoxyacetic acid (0.65 g) m.p. 170-72°C; NMR: 3.1-3.5[m, 4H, $CH_2NHCH_2$]; 3.95-4.05[m, 2H, $OCH_2CHOH$]; 4.20-4.40[m, 3H, CHOH and $NHCH_2CH_2O$]; 4.60[s, 2H, $OCH_2CO$]; 6.53[m, 1H, $H_2$ thienyl]; 6.78[m, 1H, $H_4$ thienyl]; 6.83-6.98-[m, 4H, $C_6H_4$]; 7.35[m, 1H $H_5$ thienyl]: microanalysis: found: C, 55.5; H, 5.9; N, 3.8; S, 8.7%; $C_{17}H_{21}NO_6S$ requires: C, 55.6; H, 5.7; N, 3.8; S, 8.7%.

## Example 10

4-[2-Hydroxy-3-(3-thienyloxy)propylamino)ethoxy]phenoxyacetamide.

Methyl 4-[2-(2-hydroxy-3-(3-thienyloxy)propylamino)ethoxy]phenoxyacetate (1.0 g) was added to a cold solution of methanol (180 ml) pre-saturated with gaseous ammonia. The mixture was stirred for 4 hours, and then the solvent and reagent were removed by evaporation. The residue was dissolved in fresh methanol which was then removed by evaporation. A colourless solid was obtained which was dissolved in a mixture of methanol and dichloromethane and acidified to pH 3 with ethereal hydrogen chloride. The solvent was evaporated to give a white solid which was recrystallised from methanol (120 ml) to give 4-[2-(2-hydroxy-3-(3-thienyloxy)propylamino)ethoxy]phenoxyacetamide hydrochloride (0.61 g) m.p. 224-26°C; NMR: DMSO: 3.0-3.45[m, 4H, $CH_2NH_2CH_2$]; 3.90-4.04[m, 2H, $OCH_2CHOH$]; 4.18-4.32[m, 3H, CHOH plus $CH_2CH_2OC_6H_4$]; 4.35[s, 2H, $OCH_2CO$]; 5.87[m, 1H, OH]; 6.52[m, 1H, $H_2$ thienyl]; 6.8[m, 1H, $H_4$ thienyl]; 6.86-6.98[m, 4H, $C_6H_4$]; 7.32[s, 2H, $CONH_2$] ;7.45[m, 1H, $H_5$ thienyl]; 9.05[s, 2H, $NH_2$]: microanalysis: found: C, 50.6; H, 5.5; N, 6.9; S, 8.2; Cl 8.7%; $C_{17}H_{22}N_2O_5S.HCl$ requires: C, 50.7; H, 5.7; N, 7.0; S, 8.0; Cl, 8.8%.

**Examples 11-16**

Using a procedure similar to that described in Example 10, but using the appropriate amine, the following compounds were obtained. In general the product was purified on a K60 silica column using 8% methanol in dichloromethane as eluent. In each case the product was isolated as the hydrochloride salt.

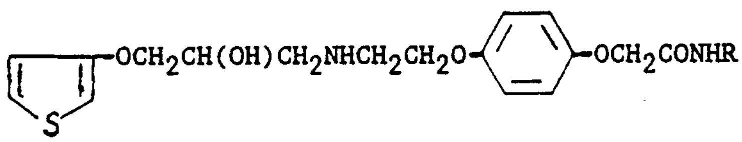

| Example | R | m.p. (°C) | recrystallisation solvent |
|---|---|---|---|
| 11 | $-CH_3$ | 199-201 | methanol/methyl acetate |
| 12 | $-C_2H_5$ | 195-96.5 | methanol/methyl acetate |
| 13 | -cyclopropyl | 190-92 | methanol |
| 14 | $-CH_2-C{\equiv}CH$ | 171-73 | methanol/methyl acetate |
| 15 | 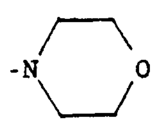 | 159-61 | methanol/methyl acetate/ ether |
| 16 | $-CH_2-CH{=}CH_2$ | 187-89 | methanol/methyl acetate |

**Spectroscopic and Analytical Data**

Example 11 [DMSO]

2.62-2.68[d, 3H, $NHCH_3$]; 3.0-3.45[m, 4H, $CH_2NH_2CH_2$]; 3.90-4•03[m, 2H, $OCH_2CHOH$]; 4.16-4.35[m, 3H, CHOH and $CH_2CH_2O$]; 4.40[s, 2H, $OCH_2CO$; 5.82-5.92[m, 1H, CHOH]; 6.62[m, 1H, $H_2$ thienyl]; 6.79[m, 1H, $H_4$ thienyl]; 6.88-7.00[m, 4H, $C_6H_4$]; 7.44[m, 1H, $H_5$ thienyl]; 8.0[s, H, $NHCH_3$]; 9.07[s, 2H, $NH_2$]: microanalysis: found: C,51.9; H,5.9; N,6.7; S,7.7; Cl,8.7%; $C_{18}H_{24}N_2O_5S$.HCl requires: C,51.9; H,6.0; N,6.7; S,7.7; Cl,8.5%: M/S: $381(M+H)^+$.

Example 12

1.10[t, 3H, $CH_2CH_3$]; 3.13-3.52[m, 6H, $CH_2NHCH_2$ and $CH_2CH_3$]; 3.96-4.10[m, 2H, $OCH_2CHOH$]; 4.22-4.38[m, 3H, CHOH and $NCH_2CH_2O$]; 4.45[s, 2H, $OCH_2CO$]; 6.53[m, 1H, $H_2$ thienyl]; 6.79[m, 1H, $H_4$ thienyl]; 6.96[s, 4H, $C_6H_4$]; 7.35[m, 1H, $H_5$ thienyl]: microanalysis: found: C,53.1; H,6.4; N,6.4; S,7.6; Cl,7.9%; $C_{19}H_{26}N_2O_5S$.HCl requires: C,53.0; H,6.3; N,6.5; S,7.4; Cl,8.3%: M/S:$395(M+H)^+$.

Example 13

0.45-0.74[m, 4H, $CHCH_2CH_2$]; 2.70[m, 1H, NHCH]; 3.05-3.48[m, 4H, $CH_2NHCH_2$]; 3.95-4.05[m, 2H, $OCH_2CHOH$]; 4.25[m, 3H, CHOH and $CH_2CH_2O$]; 4.39[s, 2H, $OCH_2CO$]; 6.57[m, 1H, $H_2$ thienyl]; 6.78[m, 1H, $H_4$ thienyl]; 6.86-6.98[m, 4H, $C_6H_4$]; 7.39[m, 1H, $H_5$ thienyl]: microanalysis: found: C,54.1; H,5.8; N,6.0; S,7.5; Cl,8.0%; $C_{20}H_{26}N_2O_5S$.HCl requires: C,54.2; H,6.1; N,6.3; S,7.2; Cl,8.0%: M/S: $407(M+H)^+$.

Example 14

2.80[t, 1H, ≡CH]; 3.10-3.50[m, 4H, $CH_2NHCH_2$]; 3.95-4.05[m, 4H, $CH_2C$≡ and $OCH_2CHOH$]; 4.22-4.35-[m, 3H, CHOH and $NCH_2CH_2O$]; 4.48[s, 2H, $OCH_2CO$]; 6.55[m, 1H, $H_2$ thienyl]; 6.78[m, 1H, $H_4$ thienyl]; 6.95[s, 4H, $C_6H_4$]; 7.35[m, 1H, $H_5$ thienyl]: microanalysis: found: C,53.4; H,5.4; N,6.2; S,8.4; Cl,8.1; $H_20$, 1.4%; $C_{20}H_{24}N_2O_5S$.HCl. $0.4H_20$ requires: C,53.6; H,5.8; N,6.3; S,7.2; Cl,7.9; $H_20$, 1.6%: (sulphur analysis performed on low sample weight) M/S:$405(M+H)^+$.

Example 15

3.10-3.38[m, 2H, $CHOHCH_2NH$]; 3.40-3.68[m, 10H, $NHCH_2CH_2O$ and morpholine protons]; 3.96-4.08[m, 2H, $OCH_2CHOH$]; 4.20-4.37[m, 3H CHOH and $NHCH_2CH_2O$]; 4.75[s, 2H, $OCH_2CO$]; 6.54[m, 1H, $H_2$ thienyl]; 6.78[m, 1H, $H_4$ thienyl]; 6.86-6.97[m, 4H, $C_6H_4$]; 7.36[m, 1H, $H_5$ thienyl]: microanalysis: found: C,53.0; H,6.2; N,5.8; S,6.8; Cl,7.1%; $C_{21}H_{28}N_2O_6S$.HCl requires: C,53.3; H,6.1; N,5.9; S,6.8; Cl,7.5%: M/S $437(M+H)^+$.

Example 16

3.06-3.50[m,4H,$CH_2NHCH_2$]; 3.75-3.86[d,2H,$CH_2CH=$]; 3.9-4.08[m,2H, $OCH_2CHOH$]; 4.18-4.36[m,3H, CHOH and $NCH_2CH_2O$]; 4.48[s,2H, $OCH_2CO$]; 5.00-5.18[m,2H,$CH=CH_2$]; 5.71-5.93[m,1H,$CH=CH_2$]; 6.56-[m,1H,$H_2$ thienyl]; 6.78[m,1H,$H_4$ thienyl]; 6.96[s,4H,$C_6H_4$]; 7.38,[m,1H,$H_5$ thienyl]: microanalysis: found: C,54.0; H,5.9; N,6.2; S,7.2; Cl,8.1%; $C_{20}H_{26}N_2O_5S$.HCl requires: C,54.2; H,6.1; N,6.3; S,7.2; Cl,8.0%: M/S $407(M+H)^+$.

Example 17

N-(4-Pyridylmethyl)-4-[2-(2-hydroxy-3-(3-thienyloxy)propylamino)ethoxy]phenoxyacetamide.

Potassium t-butoxide (0.53 g) was added to a solution of 4-[2-(2-hydroxy-3-(3-thienyloxy)propylamino)-ethoxy]phenol (1.3 g) in dimethoxyethane (110 ml) and the mixture was stirred for 10 minutes. To this

mixture was added N-(4-pyridylmethyl)chloroacetamide (1.1 g) and stirring was continued for 5 days. The solvent was evaporated and the residue purified by chromatography on K60 silica (90 g) using methanol in dichloromethane as eluent (6% methanol grading to 8% methanol). The desired product was dissolved in dichloromethane (100 ml) and acidified to pH 3 with ethereal hydrogen chloride. The solvent was evaporated and the residue was recrystallised from a mixture of ethanol and propan-2-ol to give, after drying at 60°C under high vacuo (over $P_2O_5$), N-(4-pyridylmethyl)-4-[2-(2-hydroxy-3-(3-thienyloxy)-propylamino)ethoxy]phenoxyacetamide dihydrochloride, 0.5 $H_2O$ (0.7 g), m.p. 129-31°C, hygroscopic solid: NMR: 3.05-3.46[m, 4H, $CH_2NHCH_2$]; 3.95-4.05[m, 2H, $OCH_2CHOH$]; 4.22-4.35[m, 3H, CHOH and $CH_2CH_2OC_6H_4$]; 4.58[s, 2H, $OCH_2CO$]; 4.63[s, 2H, $CH_2C_5H_4N$]; 6.60[m, 1H, $H_2$ thienyl]; 6.80[m, 1H, $H_4$ thienyl]; 6.94-7.03[m, 4H, $C_6H_4$]; 7.4[m, 1H, $H_5$ thienyl]; 7.85-8.88[m, 4H, $C_5H_4N$]: microanalysis: found: C, 51.2; H, 5.6; N, 7.7; S, 6.0; Cl, 12.7; $H_2O$, 4.3%; $C_{23}H_{27}N_3O_5S$.2HCl 0.5 $H_2O$: requires: C, 51.2; H, 5.7; N, 7.8; S, 5.9; Cl, 13.1; $H_2O$, 1.7%.

## Example 18

4-[2-(2-Hydroxy-3-(3-thienyloxy)propylamino)ethoxy]phenoxyacetohydroxamic acid.

A mixture of potassium hydroxide (0.68 g) and hydroxylamine hydrochloride (0.68 g) in methanol (20 ml) was stirred for 30 minutes. To this solution was added methyl 4-[2-(2-hydroxy-3-(3-thienyloxy)-propylamino)ethoxy]phenoxyacetate (0.95 g) and the resultant suspension was stirred for 6 days. Acetic acid (0.3 ml) was added, and the mixture was stirred for a further 30 minutes. The crude product was collected by filtration, washed with methanol and was recrystallised from methanol (110 ml) to give 4-[2-(2-hydroxy-3-(3-thienyloxy)propylamino)ethoxy]phenoxyacetohydroxamic acid, hydrochloride, (0.42 g) m.p. 187-89°C: NMR: DMSO: 3.00-3.42[m, 4H, $CH_2NH_2CH_2$]; 3.90-4.00[m, 2H, $OCH_2CHOH$]; 4.15-4.30[m, 3H, CHOH and $CH_2CH_2OC_6H_4$]; 4.38[s, 2H, $OCH_2CO$]; 5.85[d, 1H, CHOH]; 6.60[m, 1H, $H_2$ thienyl]; 6.78[m, 1H, $H_4$ thienyl]; 6.9[s, 4H, $C_6H_4$]; 7.42[m, 1H, $H_5$ thienyl]; 8.9[s, 1H, NHOH]; 9.05[s, 2H, $NH_2$]; 10.78[s, 1H, NHOH]: microanalysis; found: C, 48.8; H, 5.5; N, 6.4; S, 7.5; Cl, 8.8%; $C_{17}H_{22}N_2O_6S$.HCl requires: C, 48.8; H, 5.5; N, 6.7; S, 7.5; Cl, 8.5%.

## Example 19

0-Methyl-4-[2-(2-hydroxy-3-(3-thienyloxy)propylamino)ethoxy]phenoxyacetohydroxamate

A mixture of 0-methylhydroxylamine hydrochloride (1.75g) and potassium hydroxide (1.45g) in methanol (50ml) was stirred at ambient temperature for 10 minutes. Methyl 4-[2-(2-hydroxy-3-(3-thienyloxy)-propylamino)ethoxy]phenoxyacetate (1.22g) was added and stirring was continued for 14 days. Acetic acid (0.8ml) was added, the mixture was filtered, and the filtrate was evaporated. The residue (0.6g) was purified on a short silica column using 10% methanol in dichloromethane as eluant. The product (0.3g) was recrystallised twice from methanol to yield O-methyl-4-[2-(2-hydroxy-3-(3-thienyloxy)propylamino)ethoxy]-phenoxyacetohydroxamate hydrochloride (100mg); m.p. 162-64°C; NMR: 3.07-3.46[m,4H, $CH_2NHCH_2$]; 3.65[s,3H,$CH_3$]; 3.93-4.06[m,2H, $OCH_2CHOH$]; 4.18-4.32[m,3H,CHOH and $NHCH_2CH_2O$]; 4.46-[s,2H,$OCH_2CO$]; 6.57[m,1H,$H_2$ thienyl]; 6.78 [m,1H,$H_4$ thienyl]; 6.95[s,4H,$C_6H_4$]; 7.38[m,1H,$H_5$ thienyl]: microanalysis: found: C,49.8; H,5.8; N,6.4; S,7.6; Cl, 8.1%; $C_{18}H_{24}N_2O_6S$.HCl requires: C,49.9; H, 5.8; N, 6.5; S, 7.4; Cl, 8.2%.

## Example 20

(S)-Methyl 4-[2-(2-hydroxy-3-(3-thienyloxy)propylamino)ethoxy]phenoxyacetate

Potassium t-butoxide (0.63g) and (S)-4-[2-(2-hydroxy-3-(3-thienyloxy)propylamino)ethoxy]phenol (1.5g) were stirred in dimethoxyethane (100ml) and tetrahydrofuran (75ml) for 10 minutes. Methyl bromoacetate (0.8g) was added and the mixture stirred for 16 hours. The mixture was evaporated and the residue purified

by chromatography on K60 silica (80g) using 8% methanol in dichloromethane as eluant. The product (1.4g) was converted into the hydrochloride salt which was recrystallised from methyl acetate (20ml) and methanol (15ml) to give (S)-methyl 4-[2-(2-hydroxy-3-(3-thienyloxy)propylamino)ethoxy]phenoxyacetate hydrochloride (0.62g): m.p. 154-56 °C; NMR: 3.02-3.45[m,4H,$CH_2NHCH_2$]; 3.70[s,3H, $OCH_3$]; 3.92-4.00[m,2H, $OCH_2CHOH$]; 4.16-4.30[m,3H, CHOH and $NHCH_2CH_2O$]; 4.74 [s,2H,$OCH_2CO$]; 6.61[m,1H,$H_2$ thienyl]; 6.79 [m,1H,$H_4$ thienyl]; 6.86-6.98[m,4H,$C_6H_4$]; 7.44[m,1H,$H_5$ thienyl]. microanalysis: found: C,51.6; H, 5.7; N, 3.3; S, 7.7; Cl, 8.4%: $C_{18}H_{23}NO_6S.HCl$ requires C,51.7; H,5.8; N, 3.4; S, 7.7; Cl, 8.5%: $[\alpha]_D$ = -12.5° (c=1, methanol).

The starting material was prepared as follows:

A mixture of (S)-3-thienylglycidyl ether (2.1g) and 4-(2-aminoethoxy)phenol (4.05g) in propan-2-ol (140ml) was heated to reflux for 2 hours. The mixture was evaporated and applied to a K60 silica column (70g) using 6% methanol in dichloromethane as eluant. The crude product (2.7g) which was obtained was recrystallised from ethyl acetate to give (S)-4-[2-(2-hydroxy-3-(3-thienyloxy)propylamino)ethoxy]phenol (2.4g): m.p. 139-41 °C: NMR: 3.04-3.43[m,4H,$CH_2NHCH_2$]; 3.92-4.05[m,2H, $OCH_2CHOH$]; 4.12-4.31[m,3H, CHOH and $NHCH_2CH_2O$]; 6.56[m,1H,$H_2$ thienyl] 6.68-6.88[m,5H,$H_4$ thienyl plus $C_6H_4$]; 7.38[m,1H,$H_5$ thienyl].

(S)-3-Thienylglycidyl ether was prepared as described below.

(S)-1-(3-Thienyloxy)propan-2,3-diol (8.7g) was dissolved in pyridine (100ml) and cooled to -5 °C. 2-Naphthalenesulphonyl chloride (11.0g) was added in portions such that the temperature remained below -5 °C. The mixture was then stirred at -5 °C for 1 hour and at +5 °C for 16 hours. The cold solution was diluted with ethyl acetate (250ml) and acidified with a solution of sulphuric acid (50ml) in water (200ml). The mixture was separated and the aqueous solution was extracted with ethyl acetate. The ethyl acetate extract was dried and then evaporated to give a gum (15.5g) which consisted of an impure mixture of (R)-1-(2-naphthylsulphonyloxy)-3-(3-thienyloxy)propan-2-ol and (S)-2-(2-naphthylsulphonyloxy)-3-(3-thienyloxy)-propan-2-ol (9:1). These were separated by chromatography on K60 silica using 6% ether in dichloromethane as eluant. The principal product (4.0g), which was slightly impure, was further purified by chromatography on K60 silica (95g) using 22% acetone in cyclohexane as eluant. Thus was obtained (R)-1-(2-naphthylsulphonyloxy)-3-(3-thienyloxy)propan-2-ol (7.5g), as an oil: NMR: $CDCl_3$: 3.93-4.02-[m,2H,$OCH_2CHOH$]; 4.16-4.30[m,3H, $CHOHCH_2OSO_2$]; 6.18[m,1H,$H_2$ thienyl]; 6.59[m,1H,$H_4$ thienyl]; 7.10-[m,1H,$H_5$ thienyl]; 7.56-8.53 [m,7H,$C_{10}H_7$]; [Addition of trichloroacetyl isocyanate moved the CHOH proton to 5.35, proving that the sulphonate was attached to the primary OH].

To a solution of (R)-1-(2-naphthylsulphonyloxy)-3-(3-thienyloxy)propan-2-ol (5.5g) in dimethylsulphoxide (20ml) was added a solution of sodium hydroxide (1.5g) in water (8ml). The mixture was stirred for 15 minutes, diluted with water (50ml) and extracted with ethyl acetate (4 x 20ml). The extracts were dried ($MgSO_4$) and evaporated to give the crude product (2.9g) which was purified by chromatography on K60 silica (70g) using 14% ethyl acetate in hexane as eluant. Thus was obtained (S)-3-thienylglycidyl ether (2.1g) as an oil. NMR: $CDCl_3$: 2.68-2.94[m,2H,$CHCH_2OCH$]; 3.26-3.38[m,1H,CH]; 3.86-4.25[m,$OCH_2CHOH$]; 6.30[m,1H,$H_2$ thienyl]; 6.78 [m, 1H; $H_4$ thienyl]; 7.18[m,1H,$H_5$ thienyl].

(S)-1-(3-Thienyloxy)propan-2,3-diol was prepared as follows:

A mixture of (R)-(-)-2,2-dimethyl-1,3-dioxolane-4-methanol (92.4g), dimethoxyethane (1.5 litres) and potassium t-butoxide (78.4g) was stirred under an argon atmosphere until most of the potassium salt had dissolved. Potassium iodide (0.63g), copper oxide (28g) and 3-bromothiophene (65ml) were added and the mixture was stirred at 90 °C for 10 days. The reaction mixture was cooled, diluted with ether (200ml) and filtered to remove the inorganic salts. The filtrate was evaporated under high vacuum to give a thick brown oil (101g). The oil was purified by chromatography on K60 silica (285g) using 4% ethyl acetate in dichloromethane as eluant. Thus was obtained (S)-1-(3-thienyloxy)-2,3-0-isopropylidene propan-2,3-diol (77g) as an oil. A portion of this oil (41g) was dissolved in 80% acetic acid (250ml) and heated on the steam bath for 40 minutes. The reagent was removed by evaporation, last traces being co-evaporated with toluene. The residue (35g) slowly crystallised and was recrystallised from ethyl acetate, to give (S)-1-(3-thienyloxy)propan-2,3-diol (22.5g) m.p. 71-73 °C NMR $CDCl_3$ 400MHz: 2.0[s,1H,OH]; 2.55[s,1H,OH]; 3.72-3.85[m,2H,$CH_2OH$]; 4.04-4.11[m,3H, $OCH_2CHOH$]; 6.30[m,1H,$H_2$ thienyl]; 6.77[m,1H,$H_4$ thienyl]; 7.19-[m,1H,$H_5$ thienyl]: [Addition of TFAE ((R)-(-)-2,2,2-trifluoro-1-(9-anthryl)ethanol) did not produce any separation of signals].

**Example 21**

(S)-N-Methyl 4-[2-(2-hydroxy-3-(3-thienyloxy)propylamino)ethoxy]phenoxyacetamide.

A solution of (S)-methyl 4-[2-(2-hydroxy-3-(3-thienyloxy)propylamino)ethoxy]phenoxyacetate (1.23g) was dissolved in methanol (100ml) and a 33% solution of methylamine in ethanol (50ml). The solution was kept at ambient temperature for 24 hours and then the solvent and reagent were evaporated. The residue (1.1g) was dissolved in a mixture of dichloromethane and methanol and acidified to pH3 with ethereal hydrogen chloride. The solvent was evaporated and replaced with methanol which was again evaporated. The crude product which remained (1.3g) was recrystallised from methanol (40ml) to give (S)-N-methyl 4-[2-(2-hydroxy-3-(3-thienyloxy)propylamino)ethoxy]phenoxyacetamide hydrochloride (1.02g) m.p. 200-1 °C: 25[α]$_D$=-12.8° (c=0.5; methanol): NMR (DMSO): 2.66[d,3H, $NHCH_3$]; 3.00-3.45[m,4H, $CH_2NHCH_2$]; 3.94-4.05-[d,2H, $OCH_2CHOH$]; 4.18-4.36[m,3H,CHOH and $CH_2CH_2O$]; 4.41[s,2H,$OCH_2CO$]; 5.90[d,1H,CHOH]; 6.64-[m,1H,$H_2$ thienyl]; 6.80 [m,1H,$H_4$ thienyl]; 6.96[s,4H,$C_6H_4$]; 7.46[m,1H,$H_5$ thienyl]; 8.03[m,1H, $NHCH_3$]; 9.1-[s,1H,NH]: microanalysis: found: C, 51.6; H, 6.0; N, 6.6; S, 7.5; Cl, 8.4%: $C_{18}H_{24}N_2O_5S$.HCl requires C, 51.9; H, 6.0; N, 6.7; S, 7.7; Cl, 8.5%.

**Example 22**

(S)-4-[2-(2-Hydroxy-3-(3-thienyloxy)propylamino)ethoxy]phenoxyacetamide

(S)-Methyl 4-[2-(2-hydroxy-3-(3-thienyloxy)propylamino)ethoxy]phenoxyacetate (32g) was added to a cold solution of methanol (1.5 litres) pre-saturated with ammonia. The mixture was stirred for 4 hours and then the solvent and reagent were evaporated (aided by co-evaporation with methanol). The residue was dissolved in a mixture of warm methanol (1.5 litres) and dichloromethane (1 litre) and acidified to pH3 with ethereal hydrogen chloride. The mixture was evaporated and the crude product (34g) recrystallised from methanol (4 litres) to give (S)-4-[2-(2-hydroxy-3-(3-thienyloxy)propylamino)ethoxy]phenoxyacetamide hydrochloride (27.8g) m.p. 226.2 °C (by DSC) NMR: 3.05-3.50[m,4H, $CH_2NHCH_2$]; 3.95-4.00[m,2H, $OCH_2CHOH$]; 4.16-4.34 [m,3H, CHOH and $NCH_2CH_2O$]; 4.40[s,2H, $OCH_2CO$]; 6.61[m,1H, $H_2$ thienyl]; 6.82-[m,2H, $H_4$ thienyl]; 6.97[s,4H,$C_6H_4$]; 7.44[m,1H,$H_5$ thienyl] microanalysis found: C, 50.7, H,5.5; N, 6.8; S, 8.1, Cl, 8.8%: $C_{17}H_{22}N_2O_5S$.HCl requires: C,50.8; H,5.5; N, 7.0; S, 8.0; Cl, 8.8%: M/S + ve FAB 367 $(M+H)^+$; 25 [α]$_D$ = -14.0° (c=0.1; methanol).

**Example 23**

3-[2-(4-(2-Ethoxyethoxy)phenyl)ethylamino]-1-(3-thienyloxy]propan-2-ol

A mixture of 3-thienylglycidyl ether (0.5g) and 2-(4-(2-ethoxyethoxy)phenyl)ethylamine [EP-A-254856] (1.0g) in propan-2-ol (25ml) was heated to reflux for 3 days. The solvent was evaporated, and the residue purified by chromatography on K60 silica (75g) using 10% methanol in dichloromethane as eluant. The crystalline product which was obtained (0.15g) was dissolved in a mixture of dichloromethane and methanol and acidified to pH3 with ethereal hydrogen chloride. The solvent was evaporated and the residue recrystallised from methyl acetate (25ml) plus a few drops of methanol. Thus was obtained 3-[2-(4-(2-ethoxyethoxy)phenyl)ethylamino]-1-(3-thienyloxy)propan-2-ol (0.105g) m.p. 166-68 °C; NMR: 1.13-[t,3H,$CH_2CH_3$]; 2.83-3.01 [m,2H, $CH_2C_6H_4$]; 3.01-3.27[m,4H, $CH_2NHCH_2$]; 3.44-3.58[q,2H, $CH_2CH_3$]; 3.64-3.73[pr.d,2H, $OCH_2CH_2O$]; 3.9-4.02[m,2H,$PhOCH_2$]; 4.02-4.10 [pr.d,2H, $OCH_2CH_2O$]; 4.13-4.27 [m,1H,CHOH] 6.58[m,1H, $H_2$ thienyl]; 6.78[m,1H,$H_4$ thienyl]; 6.85-7.23 [m,4H, $C_6H_4$]; 7.40[m,1H,$H_5$ thienyl]; microanalysis: found: C, 57.1; H, 6.5; N, 3.5; S, 8.1%: $C_{19}H_{27}NO_4S$.HCl requires: C, 56.8; H, 7.0; N, 3.5; S, 8.0%.

**Example 24**

(S)-Methyl 4-[2-(2-hydroxy-3-(3-thienyloxy)propylamino)ethoxy]phenoxyacetate.

A mixture of methyl 4-[2-(2-hydroxy-3-(3-thienyloxy)propylamino)ethoxy]phenoxyacetate (4.55 g), (-)-di-p-toluoyltartaric acid (4.6 g) in methanol (40 ml) was evaporated by boiling to give a final volume of about 10 ml. Methyl acetate (50 ml) was added and the mixture was again concentrated to 25 ml volume. The mixture was left at ambient temperature for 18 hours. The solid which had formed was collected and recrystallised from methanol to give (S)-methyl-4-[2-(2-hydroxy-3-(3-thienyloxy)propylamino)ethoxy]-phenoxyacetate (-)-hydrogen-di-p-toluoyl tartrate (3.1 g); m.p. 145-147°C; 25[α]$_D$, = -78.3° (c = 1.1; methanol); microanalysis, found: C, 59.3; H, 5.6; N, 1.8; S, 4.2%; $C_{38}H_{41}NO_{14}S$ requires: C, 59.5; H, 5.3; N, 1.8; S, 4.2%.

(S)-Methyl 4-[2-(2-hydroxy-3-(3-thienyloxy)propylamino)ethoxy]phenoxyacetate (-)-hydrogen-di-p-toluoyl tartrate (3.0 g) was partitioned between 5% w/v sodium hydrogen carbonate solution (100 ml) and dichloromethane (150 ml). The organic layer was separated, dried ($MgSO_4$) and the solvent was evaporated. The residual solid was crystallised from methanol to give (S)-methyl-4-[2-(2-hydroxy-3-(3-thienyloxy)-propylamino)ethoxy]phenoxyacetate (1.4 g), m.p. 94-96° C; 23[α]$_D$, = -5° (c = 0.99; methanol); microanalysis: found: C, 56.7; H, 5.9; N, 3.7; S, 8.5%; $C_{18}H_{23}NO_6S$ requires: C, 56.7; H, 6.0; N, 3.7; S, 8.4%.

A solution of (S)-methyl 4-[2-(2-hydroxy-3-(3-thienyloxy)propylamino)ethoxy]phenoxyacetate (0.175g) in methyl acetate (5ml) was heated with a solution of ether saturated with hydrogen chloride. The precipitated solid was crystallised from a mixture of methanol and methyl acetate to give (S)-methyl 4-[2-(2-hydroxy-3-(3-thienyloxy)propylamino)ethoxy]phenoxyacetate hydrochloride (0.162g), m.p. 154-155° C; 23[α]$_D$= -11.3° (c=0.97; methanol): microanalysis: found: C,51.7; H,5.3; N,3.2; Cl,8.4; S,7.6%; $C_{18}H_{23}NO_6S$.HCl requires: C,51.7; H,5.7; N,3.4; Cl,8.5; S,7.7%.

## Example 25

(S)-4-[2-(2-Hydroxy-3-(3-thienyloxy)propylamino)ethoxy]phenoxyacetic acid.

A mixture of (S)-methyl 4-[2-(2-hydroxy-3-(3-thienyloxy)propylamino)ethoxy]phenoxyacetate (1.0 g) in methanol (20 ml) and water (1.23 ml) containing sodium hydroxide (0.123 g) was left at ambient temperature for 3 hours. The solvent was removed by evaporation. The residûal solid was dissolved in distilled water and the pH adjusted to 5.25. The solid was collected and washed with distilled water to give (S)-4-[2-(2-hydroxy-3-(3-thienyloxy)propylamino)ethoxy]phenoxyacetic acid hemihydrate (0.82 g), m.p. 176-177°C; 23[α]$_D$, = -6.6°C(c= 1.0; DMSO); microanalysis: found: C, 54.2; H, 6.0; N, 3.8; S, 8.6; $H_2O$, 2.6%; $C_{17}H_{21}NO_6S.0.5\ H_2O$ requires; C, 54.3; H, 5.9; N, 3.7; S, 8.5; $H_2O$, 2.4%.

A solution of (S)-4-[2-(2-hydroxy-3-(3-thienyloxypropylamino)ethoxy]phenoxyacetic acid hemihydrate (0.175g) in methanol (10ml) was heated with a solution of ether saturated with hydrogen chloride. Ether (60ml) was added. The precipitated solid was crystallised from a mixture of methanol and ethyl acetate to give (S)-4-[2-(2-hydroxy-3-(3-thienyloxy)propylamino)ethoxy]phenoxyacetic acid hydrochloride (0.125g), m.p. 172° C; 23[α]$_D$ = -12.4° (c=1.01; methanol): microanalysis, found: C,50.6; H,5.2; N,3.3; Cl,8.7; S,8.0%; $C_{17}H_{21}NO_6S$.HCl requires: C,50.6; H,5.5; N,3.5; Cl,8.8; S,7.9%.

## Example 26

N-(3-Hydroxypropyl)-4-[2-(2-hydroxy-3-(3-thienyloxy)propylamno)ethoxy]phenoxyacetamide.

Methyl 4-[2-(2-hydroxy-3-(3-thienyloxy)propylamino)ethoxy]phenoxyacetate (0.2g) and 3-aminopropan-1-ol (0.5ml) were heated together on the steam-bath for 3 hours. The mixture was cooled, dissolved in methanol (5ml) and treated with a solution of ether saturated with hydrogen chloride. Excess solvent was evaporated under reduced pressure. The residue was heated with water (5ml). The resulting solid was filtered and washed with cold water and acetone and crystallised from methanol and ethyl acetate to give N-(3-hydroxypropyl)-4-[2-(2-hydroxy-3-(3-thienyloxy)propylamino)ethoxy]phenoxyacetamide hydrochloride (0.12g), m.p. 172-174°; microanalysis: found: C,52.1; H,6.2; N,6.0; Cl,7.4: S,7.1%; $C_{20}H_{28}N_2O_6S$.HCl requires: C,52.1; H,6.3; N,6.1; Cl,7.7; S,7.0%.

**Example 27**

As stated previously, suitable pharmaceutical compositions of the compounds of the formula (I) may be obtained by standard formulation techniques.

A typical tablet formulation suitable for oral administration to warm-blooded animals comprises as active ingredient a micronised and/or milled form of a compound of formula I, or a pharmaceutically acceptable salt thereof, as defined hereinbefore (for example as described in one of the preceding Examples), and may be produced by direct compression or wet granulation followed by compression together with micronised and/or milled lactose containing a standard disintegrant and/or lubricant.

## Claims

1. A compound of the formula (I):

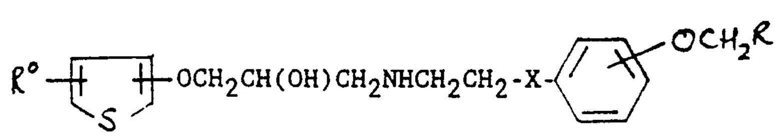

(I)

wherein
X is oxygen or is a bond;
$R^1$ is carboxy(-COOH), $C_{1-6}$alkoxycarbonyl, or a group $-CONR^2R^3$ wherein $R^2$ is hydrogen, $C_{1-6}$alkyl or $C_{3-6}$alkenyl (either group being optionally substituted by hydroxy, $C_{1-4}$alkoxy, phenoxy, carbamoyl, $C_{1-6}$alkylcarbamoyl, di-$C_{1-6}$alkylcarbamoyl, pyridyl, phenyl, chlorophenyl or chloropyridyl), $C_{3-6}$alkynyl, $C_{3-6}$cycloalkyl or $R^2$ is phenyl optionally substituted by halo, $C_{1-4}$alkyl, $C_{1-4}$alkoxy, trifluoromethyl, cyano or nitro;
$R^3$ is hydrogen, $C_{1-6}$alkyl, or a group $-OR^4$ wherein $R^4$ is hydrogen, $C_{1-6}$alkyl optionally substituted by hydroxy or $C_{1-4}$alkoxy, $C_{3-6}$alkenyl optionally substituted by hydroxy or $C_{1-4}$alkoxy, or $R^4$ is $C_{3-6}$alkynyl, phenyl, naphthyl, phenyl($C_{1-4}$)alkyl or naphthyl($C_{1-4}$)alkyl;
or $R^2$ and $R^3$ together form a $C_{4-7}$ methylene chain (wherein one methylene unit may optionally be replaced by oxygen or sulphur situated at least two carbon atoms distant from the nitrogen atom of $-NR^2R^3$) in which two adjacent methylene units may optionally be replaced by two carbon atoms of a benzene ring fused to said $C_{4-7}$ methylene chain said benzene ring optionally substituted by halo, $C_{1-4}$ alkyl, $C_{1-4}$ alkoxy, trifluoromethyl, cyano or nitro, or $R^2$ and $R^4$ together form a $C_{3-4}$ methylene chain;
or $R^1$ is $C_{1-6}$alkanoyl or a group $-(CH_2)_nOR^5$ wherein n is 1, 3 or 5 and $R^5$ is hydrogen, $C_{1-4}$alkyl or $C_{1-4}$alkanoyl;
and wherein $R^0$ is hydrogen or is chloro or fluoro substituted ortho to the aminopropoxy side-chain:
or a pharmaceutially acceptable salt thereof.

2. A compound according to claim 1 wherein the thienyloxypropylaminoethyl or thienyloxypropylaminoethoxy subs tituent and the $-OCH_2R^1$ substituent are in para-relationship.

3. A compound according to either claim 1 or claim 2 wherein $R^0$ is hydrogen.

4. A compound according to any one of claims 1 to 3 wherein X is oxygen.

5. A compound according to any one of claims 1 to 4 wherein the oxypropylamino side chain is substituted at the 3-position of the thiophene ring.

6. A compound according to any one of claims 1 to 5 wherein $R^6$ is carboxy, $C_{1-6}$alkoxycarbonyl, carbamoyl, $C_{1-6}$alkylcarbamoyl, hydroxy$C_{1-6}$alkylcarbamoyl, $c_{1-4}$alkoxy$C_{1-6}$alkylcarbamoyl, di-$C_{1-6}$alkylcarbamoyl, piperidino or morpholino.

7. A compound of the formula (IIA):

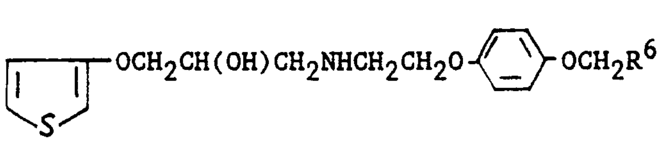

(IIA)

wherein R6 is carboxy, methoxycarbonyl, carbamoyl, methylcarbamoyl or 2-methoxyethylcarbamoyl.

8. A compound according to any one of claims 1 to 7 in laevorotatory optically active form.

9. A compound which is:
methyl 4-[2-(2-hydroxy-3-(3-thienyloxy)propylamino)ethyl]phenoxyacetate,
methyl 4-[2-(2-hydroxy-3-(3-thienyloxy)propylamino)ethoxy]phenoxyacetate,
(S)-methyl 4-[2-(2-hydroxy-3-(3-thienyloxy)propylamino)ethyl] phenoxyacetate, or
(S)-methyl 4-[2-(2-hydroxy-3-(3-thienyloxy)propylamino)ethoxy]phenoxyacetate.

10. A compound which is:
4-[2-(2-hydroxy-3-(3-thienyloxy)propylamino)ethoxy]phenoxyacetic acid,
4-[2-(2-hydroxy-3-(3-thienyloxy)propylamino)ethyl]phenoxyacetic acid,
(S)-4-[2-(2-hydroxy-3-(3-thienyloxy)propylamino)ethoxy]phenoxyacetic acid, or
(S)-4-[2-(2-hydroxy-3-(3-thienyloxy)propylamino)ethyl]phenoxyacetic acid,

11. A compound which is:
N-2-methoxyethyl-4-[2-(2-hydroxy-3-(3-thienyloxy)propylamino)ethoxy]phenoxyacetamide,
N-methyl-4-[2-(2-hydroxy-3-(3-thienyloxy)propylamino)ethoxy]phenoxyacetamide,
4-[2-(2-hydroxy-3-(3-thienyloxy)propylamino)ethoxy]phenoxyacetamide,
(S)-4-[2-(2-hydroxy-3-(3-thienyloxy)propylamino)ethoxy]phenoxyacetamide,
N-ethyl-4-[2-(2-hydroxy-3-(3-thienyloxy)propylamino)ethoxy]phenoxyacetamide or (S)-N-methyl-4-[2-(2-hydroxy-3-(3-thienyloxy)propylamino)ethoxy]phenoxyacetamide.

12. A pharmaceutical composition which comprises a compound according to any one of claims 1 to 11 and a pharmaceutically acceptable carrier therefor.

13. A process for preparing a compound of the formula (I) as defined in claim 1 or a pharmaceutically acceptable salt thereof which comprises:
(a) reacting a compound of the formula (III) with a compound of the formula (IV):

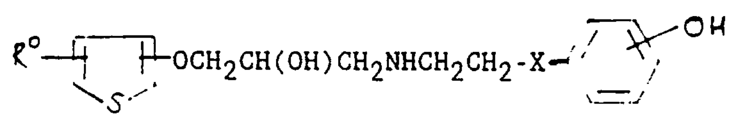

(III)

$L-CH_2-R^1$ (IV)

wherein X, $R^0$ and R1 are as defined in claim 1 and L is a displaceable group; or
(b) reacting a compound of the formula (V) with a compound of the formula (VI):

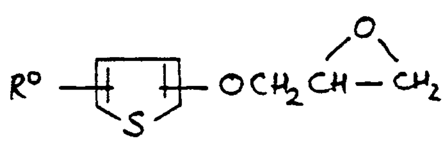

(V)

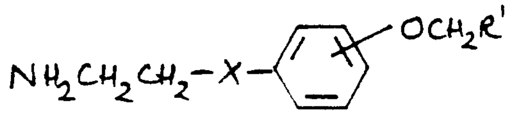

(VI)

wherein X, $R^0$ and $R^1$ are as defined in claim 1; or
(c) reducing a compound of the formula (VII):

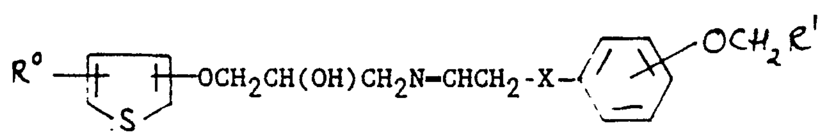

(VII)

wherein X, $R^0$ and $R^1$ are as hereinabove defined:

(d) for preparing compounds wherein $R^1$ is carbamoyl ($-CONH_2$), hydrolysing a compound of the formula (VIII):

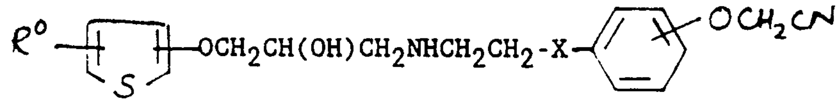

(VIII)

wherein X and $R^0$ are as defined in claim 1;

and, if desired thereafter:

(i) converting a compound of the formula (I) into another compound of the formula (I), and/or

(ii) forming a pharmaceutically acceptable salt.

14. A compound of the formula (III):

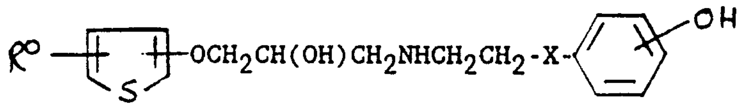

(III)

wherein $R^0$ is hydrogen or is chloro or fluoro substituted ortho to the aminopropoxy side-chain and X is a bond or is oxygen.

15. A compound of the formula (VIII):

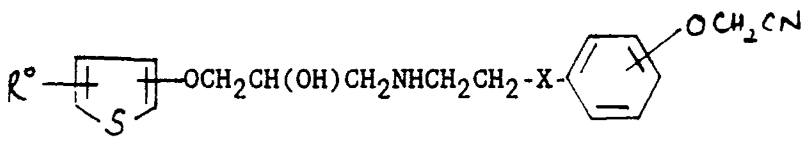

(VIII)

wherein $R^0$ is hydrogen or is chloro or fluoro substituted ortho to the aminopropoxy side-chain and X is a bond or is oxygen.

16. (S)-3-Thienylglycidyl ether of the formula:

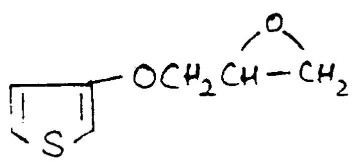

Claims for the following Contracting State: ES

1. A process for preparing a compound of the formula (I):

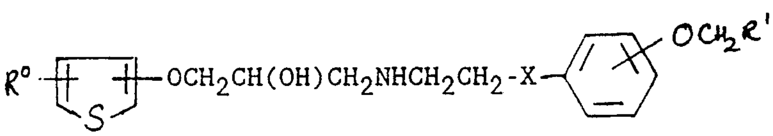

(I)

wherein

X is oxygen or is a bond;

$R^1$ is carboxy($-COOH$), $C_{1-6}$alkoxycarbonyl, or a group $-CONR^2R^3$ wherein $R^2$ is hydrogen, $C_{1-6}$alkyl or $C_{3-6}$alkenyl (either group being optionally substituted by hydroxy, $C_{1-4}$alkoxy, phenoxy, carbamoyl, $C_{1-6}$alkylcarbamoyl, di-$C_{1-6}$alkylcarbamoyl, pyridyl, phenyl, chlorophenyl or chloropyridyl), $C_{3-6}$alkynyl, $C_{3-6}$cycloalkyl or $R^2$ is phenyl optionally substituted by halo, $C_{1-4}$alkyl, $C_{1-4}$alkoxy, trifluoromethyl, cyano or nitro;

$R^3$ is hydrogen, $C_{1-6}$alkyl, or a group $-OR^4$ wherein $R^4$ is hydrogen, $C_{1-6}$alkyl optionally substituted by

hydroxy or $C_{1-4}$alkoxy, $C_{3-6}$alkenyl optionally substituted by hydroxy or $C_{1-4}$alkoxy, or $R^4$ is $C_{3-6}$alkynyl, phenyl, naphthyl, phenyl($C_{1-4}$)alkyl or naphthyl($C_{1-4}$)alkyl;
or $R^2$ and $R^3$ together form a $C_{4-7}$ methylene chain (wherein one methylene unit may optionally be replaced by oxygen or sulphur situated at least two carbon atoms distant from the nitrogen atom of $-NR^2R^3$) in which two adjacent methylene units may optionally be replaced by two carbon atoms of a benzene ring fused to said $C_{4-7}$ methylene chain said benzene ring optionally substituted by halo, $C_{1-4}$ alkyl, $C_{1-4}$ alkoxy, trifluoromethyl, cyano or nitro, or $R^2$ and $R^4$ together form a $C_{3-4}$ methylene chain;
or $R^1$ is $C_{1-6}$alkanoyl or a group $-(CH_2)_nOR^5$ wherein n is 1, 3 or 5 and $R^5$ is hydrogen, $C_{1-4}$alkyl or $C_{1-4}$alkanoyl;
and wherein $R^0$ is hydrogen or is chloro or fluoro substituted ortho to the aminopropoxy side-chain:
or a pharmaceutially acceptable salt thereof:
which process comprises:
(a) reacting a compound of the formula (III) with a compound of the formula (IV):

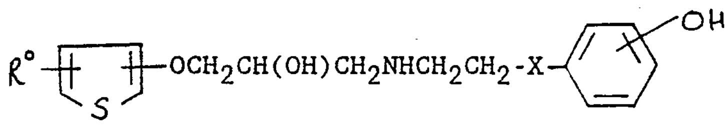
(III)

$L-CH_2-R^1$ (IV)
wherein X, $R^0$ and R1 are as defined hereinabove and L is a displaceable group; or
(b) reacting a compound of the formula (V) with a compound of the formula (VI):

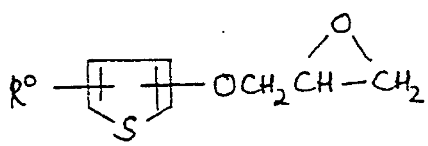
(V)

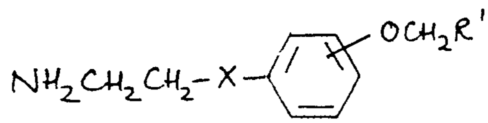
(VI)

wherein X, $R^0$ and $R^1$ are as defined hereinabove; or
(c) reducing a compound of the formula (VII):

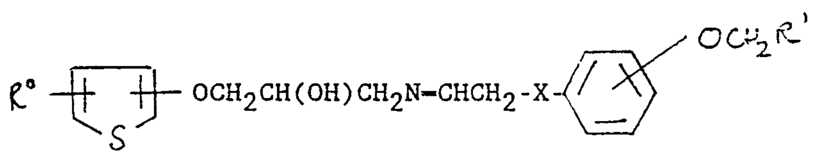
(VII)

wherein X, $R^0$ and $R^1$ are as defined hereinabove:
(d) for preparing compounds wherein $R^1$ is carbamoyl ($-CONH_2$), hydrolysing a compound of the formula (VIII):

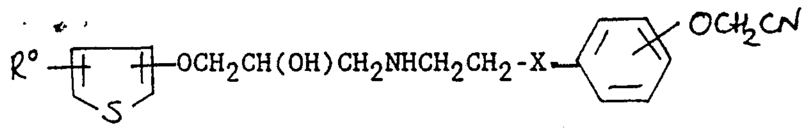

(VIII)

wherein X and $R^0$ are as defined hereinabove;
and, if desired thereafter:
(i) converting a compound of the formula (I) into another compound of the formula (I), and/or
(ii) forming a pharmaceutically acceptable salt.

2. A process according to claim 1 for preparing a compound wherein the thienyloxypropylaminoethyl or thienyloxypropylaminoethoxy substituent and the $-OCH_2R^1$ substituent are in para-relationship.

3. A process according to either claim 1 or claim 2 for preparing a compound wherein $R^0$ is hydrogen.

4. A process according to any one of claims 1 to 3 for preparing a compound wherein X is oxygen.

5. A process according to any one of claims 1 to 4 for preparing a compound wherein the oxypropylamino side chain is substituted at the 3-position of the thiophene ring.

6. A process according to any one of claims 1 to 5 for preparing a compound wherein $R^6$ is carboxy, $C_{1-6}$alkoxycarbonyl, carbamoyl, $C_{1-6}$alkylcarbamoyl, hydroxy$C_{1-6}$alkylcarbamoyl, $C_{1-4}$alkoxy$C_{1-6}$alkylcarbamoyl, di-$C_{1-6}$alkylcarbamoyl, piperidino or morpholino.

7. A process according to claim 1 for preparing a compound of the formula (IIA):

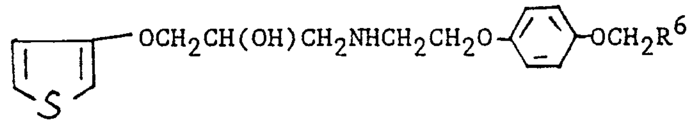

(IIA)

wherein R6 is carboxy, methoxycarbonyl, carbamoyl, methylcarbamoyl or 2-methoxyethylcarbamoyl.

8. A process according to any one of claims 1 to 7 for preparing a compound in laevorotatory optically active form.

9. A process according to claim 1 for preparing a compound which is:
methyl 4-[2-(2-hydroxy-3-(3-thienyloxy)propylamino)ethyl]phenoxyacetate,
methyl 4-[2-(2-hydroxy-3-(3-thienyloxy)propylamino)ethoxy]phenoxyacetate,
(S)-methyl 4-[2-(2-hydroxy-3-(3-thienyloxy)propylamino)ethyl]phenoxyacetate, or
(S)-methyl 4-[2-(2-hydroxy-3-(3-thienyloxy)propylamino)ethoxy]phenoxyacetate.

10. A process according to claim 1 for preparing a compound which is:
4-[2-(2-hydroxy-3-(3-thienyloxy)propylamino)ethoxy]phenoxyacetic acid,
4-[2-(2-hydroxy-3-(3-thienyloxy)propylamino)ethyl]phenoxyacetic acid,
(S)-4-[2-(2-hydroxy-3-(3-thienyloxy)propylamino)ethoxy]phenoxyacetic acid, or
(S)-4-[2-(2-hydroxy-3-(3-thienyloxy)propylamino)ethyl]phenoxyacetic acid.

11. A process according to claim 1 for preparing a compound which is:
N-2-methoxyethyl-4-[2-(2-hydroxy-3-(3-thienyloxy)propylamino)ethoxy] phenoxyacetamide,
N-methyl-4-[2-(2-hydroxy-3-(3-thienyloxy)propylamino)ethoxy]phenoxyacetamide,
4-[2-(2-hydroxy-3-(3-thienyloxy)propylamino)ethoxy]phenoxyacetamide,
(S)-4-[2-(2-hydroxy-3-(3-thienyloxy)propylamino)ethoxy]phenoxyacetamide,
N-ethyl-4-[2-(2-hydroxy-3-(3-thienyloxy)propylamino)ethoxy]phenoxyacetamide or
(S)-N-methyl-4-[2-(2-hydroxy-3-(3-thienyloxy)propylamino)ethoxy]phenoxyacetamide.

12. A process according to claim 1 wherein a compound of the formula (III) is reacted with a compound of the formula (IV), wherein L is chloro, bromo, iodo, methanesulphonyloxy or p-toluenesulphonyloxy, in the presence of a base in an organic solvent at a non-extreme temperature.

13. A process according to claim 1 wherein a compound of the formula (V) is reacted with a compound of the formula (VI) in an organic solvent, especially an alcohol, at a non-extreme temperature.

14. A process according to claim 1 wherein a compound of the formula (I) wherein $R^1$ is $C_{1-6}$alkoxycarbonyl is reacted with a compound of the formula: $NHR^2R^3$ in a solvent especially an alcohol at a non-extreme temperature, optionally in a pressure vessel.

15. A process for preparing a pharmaceutical composition which comprises bringing into association a compound of the formula (I) or pharmaceutically acceptable salt thereof and a pharmaceutically acceptable carrier.

16. The use of a compound of the formula (I) or pharmaceutically acceptable salt thereof for the

manufacture of a medicament for treating obesity.

Claims for the following Contracting State: GR

1. A process for preparing a compound of the formula (I):

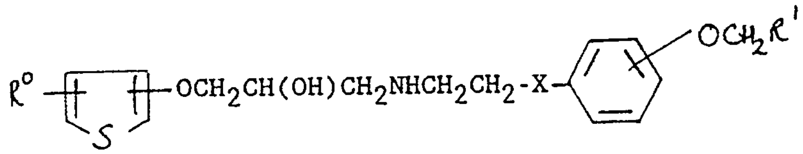

(I)

wherein

X is oxygen or is a bond;

$R^1$ is carboxy(-COOH), $C_{1-6}$alkoxycarbonyl, or a group $-CONR^2R^3$ wherein $R^2$ is hydrogen, $C_{1-6}$alkyl or $C_{3-6}$alkenyl (either group being optionally substituted by hydroxy, $C_{1-4}$alkoxy, phenoxy, carbamoyl, $C_{1-6}$alkylcarbamoyl, di-$C_{1-6}$alkylcarbamoyl, pyridyl, phenyl, chlorophenyl or chloropyridyl), $C_{3-6}$alkynyl, $C_{3-6}$cycloalkyl or $R^2$ is phenyl optionally substituted by halo, $C_{1-4}$alkyl, $C_{1-4}$alkoxy, trifluoromethyl, cyano or nitro;

$R^3$ is hydrogen, $C_{1-6}$alkyl, or a group $-OR^4$ wherein $R^4$ is hydrogen, $C_{1-6}$alkyl optionally substituted by hydroxy or $C_{1-4}$alkoxy, $C_{3-6}$alkenyl optionally substituted by hydroxy or $C_{1-4}$alkoxy, or $R^4$ is $C_{3-6}$alkynyl, phenyl, naphthyl, phenyl($C_{1-4}$)alkyl or naphthyl($C_{1-4}$)alkyl;

or $R^2$ and $R^3$ together form a $C_{4-7}$ methylene chain (wherein one methylene unit may optionally be replaced by oxygen or sulphur situated at least two carbon atoms distant from the nitrogen atom of $-NR^2R^3$) in which two adjacent methylene units may optionally be replaced by two carbon atoms of a benzene ring fused to said $C_{4-7}$ methylene chain said benzene ring optionally substituted by halo, $C_{1-4}$ alkyl, $C_{1-4}$ alkoxy, trifluoromethyl, cyano or nitro, or $R^2$ and $R^4$ together form a $C_{3-4}$ methylene chain;

or $R^1$ is $C_{1-6}$alkanoyl or a group $-(CH_2)_nOR^5$ wherein n is 1, 3 or 5 and $R^5$ is hydrogen, $C_{1-4}$alkyl or $C_{1-4}$alkanoyl;

and wherein $R^0$ is hydrogen or is chloro or fluoro substituted ortho to the aminopropoxy side-chain:

or a pharmaceutially acceptable salt thereof:

which process comprises:

(a) reacting a compound of the formula (III) with a compound of the formula (IV):

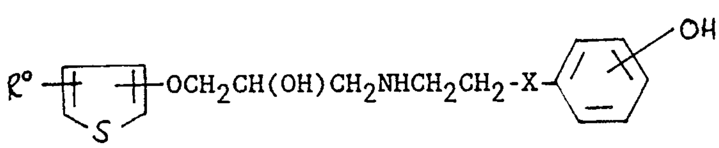

(III)

$L-CH_2-R^1$ (IV)

wherein X, $R^0$ and R1 are as defined hereinabove and L is a displaceable group; or

(b) reacting a compound of the formula (V) with a compound of the formula (VI):

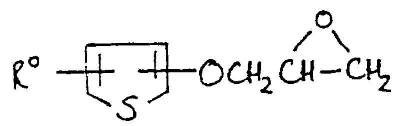

(V)

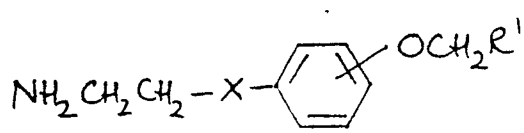

(VI)

wherein X, $R^0$ and $R^1$ are as defined hereinabove; or
(c) reducing a compound of the formula (VII):

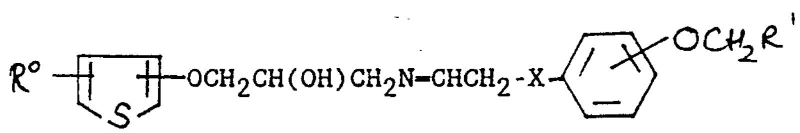

(VII)

wherein X, $R^0$ and $R^1$ are as defined hereinabove:
(d) for preparing compounds wherein $R^1$ is carbamoyl ($-CONH_2$), hydrolysing a compound of the formula (VIII):

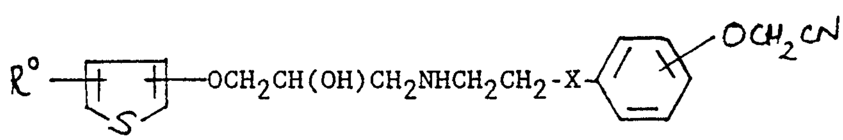

(VIII)

wherein X and $R^0$ are as defined hereinabove;
and, if desired thereafter:
(i) converting a compound of the formula (I) into another compound of the formula (I), and/or
(ii) forming a pharmaceutically acceptable salt.

2. A process according to claim 1 for preparing a compound wherein the thienyloxypropylaminoethyl or thienyloxypropylaminoethoxy substituent and the $-OCH_2R^1$ substituent are in para-relationship.

3. A process according to either claim 1 or claim 2 for preparing a compound wherein $R^0$ is hydrogen.

4. A process according to any one of claims 1 to 3 for preparing a compound wherein X is oxygen.

5. A process according to any one of claims 1 to 4 for preparing a compound wherein the oxypropylamino side chain is substituted at the 3-position of the thiophene ring.

6. A process according to any one of claims 1 to 5 for preparing a compound wherein $R^6$ is carboxy, $C_{1-6}$alkoxycarbonyl, carbamoyl, $C_{1-6}$alkylcarbamoyl, hydroxy$C_{1-6}$alkylcarbamoyl, $C_{1-4}$alkoxy$C_{1-6}$alkylcarbamoyl, di-$C_{1-6}$alkylcarbamoyl, piperidino or morpholino.

7. A process according to claim 1 for preparing a compound of the formula (IIA):

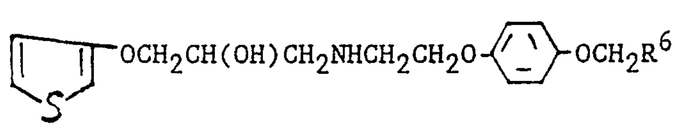

(IIA)

wherein R6 is carboxy, methoxycarbonyl, carbamoyl, methylcarbamoyl or 2-methoxyethylcarbamoyl.

8. A process according to any one of claims 1 to 7 for preparing a compound in laevorotatory optically active form.

9. A process according to claim 1 for preparing a compound which is:
methyl 4-[2-(2-hydroxy-3-(3-thienyloxy)propylamino)ethyl]phenoxyacetate,
methyl 4-[2-(2-hydroxy-3-(3-thienyloxy)propylamino)ethoxy]phenoxyacetate,
(S)-methyl 4-[2-(2-hydroxy-3-(3-thienyloxy)propylamino)ethyl]phenoxyacetate,
(S)-methyl 4-[2-(2-hydroxy-3-(3-thienyloxy)propylamino)ethoxy]phenoxyacetate,
4-[2-(2-hydroxy-3-(3-thienyloxy)propylamino)ethoxy]phenoxyacetic acid,
4-[2-(2-hydroxy-3-(3-thienyloxy)propylamino)ethyl]phenoxyacetic acid,
(S)-4-[2-(2-hydroxy-3-(3-thienyloxy)propylamino)ethoxy]phenoxyacetic acid,
(S)-4-[2-(2-hydroxy-3-(3-thienyloxy)propylamino)ethyl]phenoxyacetic acid,
N-2-methoxyethyl-4-[2-(2-hydroxy-3-(3-thienyloxy)propylamino)ethoxy]phenoxyacetamide,
N-methyl-4-[2-(2-hydroxy-3-(3-thienyloxy)propylamino)ethoxy]phenoxyacetamide,
4-[2-(2-hydroxy-3-(3-thienyloxy)propylamino)ethoxy]phenoxyacetamide,
(S)-4-[2-(2-hydroxy-3-(3-thienyloxy)propylamino)ethoxy]phenoxyacetamide,
N-ethyl-4-[2-(2-hydroxy-3-(3-thienyloxy)propylamino)ethoxy]phenoxyacetamide or
(S)-N-methyl-4-[2-(2-hydroxy-3-(3-thienyloxy)propylamino)ethoxy]phenoxyacetamide.

10. A process according to claim 1 wherein a compound of the formula (III) is reacted with a compound of the formula (IV), wherein L is chloro, bromo, iodo, methanesulphonyloxy or p-toluenesulphonyloxy, in the presence of a base in an organic solvent at a non-extreme temperature.

11. A process according to claim 1 wherein a compound of the formula (V) is reacted with a compound of the formula (VI) in an organic solvent, especially an alcohol, at a non-extreme temperature.

12. A process according to claim 1 wherein a compound of the formula (I) wherein $R^1$ is $C_{1-6}$alkoxycarbonyl is reacted with a compound of the formula: $NHR^2R^3$ in a solvent especially an alcohol at a non-extreme temperature, optionally in a pressure vessel.

13. A process for preparing a pharmaceutical composition which comprises bringing into association a compound of the formula (I) or pharmaceutically acceptable salt thereof and a pharmaceutically acceptable carrier.

14. The use of a compound of the formula (I) or pharmaceutically acceptable salt thereof for the manufacture of a medicament for treating obesity.

15. A compound of the formula (III):

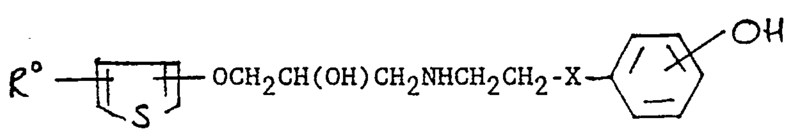

(III)

wherein $R^0$ is hydrogen or is chloro or fluoro substituted ortho to the aminopropoxy side-chain and X is a bond or is oxygen.

16. A compound of the formula (VIII):

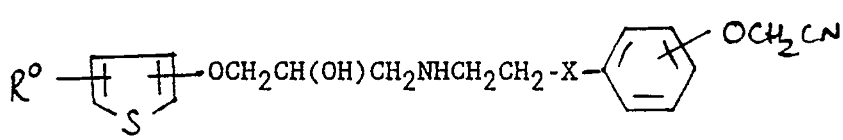

(VIII)

wherein $R^0$ is hydrogen or is chloro or fluoro substituted ortho to the aminopropoxy side-chain and X is a bond or is oxygen.

17. (S)-3-Thienylglycidyl ether of the formula: